(19) Europäisches Patentamt
European Patent Office
Office européen des brevets

(11) EP 2 491 953 B1

# (12) EUROPEAN PATENT SPECIFICATION

(45) Date of publication and mention of the grant of the patent:
**19.05.2021 Bulletin 2021/20**

(21) Application number: **10825230.5**

(22) Date of filing: **22.10.2010**

(51) Int Cl.:
*A61K 51/00* (2006.01) *A61P 35/00* (2006.01)
*A61K 103/30* (2006.01) *A61K 101/00* (2006.01)
*A61K 49/00* (2006.01) *A61K 49/18* (2006.01)
*A61K 51/02* (2006.01) *A61K 51/04* (2006.01)
*A61K 51/10* (2006.01) *A61K 51/12* (2006.01)
*G01T 1/164* (2006.01) *G01T 7/08* (2006.01)

(86) International application number:
**PCT/KR2010/007287**

(87) International publication number:
**WO 2011/049405 (28.04.2011 Gazette 2011/17)**

## (54) OPTICAL IMAGING CONTRAST AGENT, USE AND DEVICE THEREOF

KONTRASTMITTEL ZUR OPTISCHEN BILDGEBUNG SOWIE VERWENDUNG DAVON UND GERÄT DAFÜR

AGENT DE CONTRASTE D'IMAGERIE OPTIQUE, UTILISATION ET DISPOSITIF POUR CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2009 KR 20090100745**

(43) Date of publication of application:
**29.08.2012 Bulletin 2012/35**

(73) Proprietor: **Kyungpook National University Industry-
Academic Cooperation Foundation
Daegu 702-701 (KR)**

(72) Inventors:
- **YOO, Jeong-Soo
Daegu 706-140 (KR)**
- **PARK, Jeong Chan
Daegu 702-260 (KR)**

(74) Representative: **Dr. Gassner & Partner mbB
Wetterkreuz 3
91058 Erlangen (DE)**

(56) References cited:
**WO-A2-2006/024489 WO-A2-2009/045579
KR-A- 20070 091 609 KR-A- 20070 106 711
US-A- 4 874 600 US-A1- 2003 003 102**

- **S.PALAKURTHI, S.A. MCQUARRIE,M.R.SURESH: "Screening of 14C-Polyamines in the AT3B-1 Rat Prostate Tumor Model: The Search for a new PET Prostate Imaging Agent", in vivo , vol. 21 1 January 2007 (2007-01-01), 1 January 2007 (2007-01-01), pages 823-827, XP055229794, Retrieved from the Internet: URL:http://iv.iiarjournals.org/content/21/5/823.full.pdf#page=1&view=FitH [retrieved on 2015-11-19]**
- **DMITRI SOLOVIEV ET AL: "PET imaging with 11C-acetate in prostate cancer: a biochemical, radiochemical and clinical perspective", EUROPEAN JOURNAL OF NUCLEAR MEDICINE AND MOLECULAR IMAGING, SPRINGER, BERLIN, DE, vol. 35, no. 5, 13 March 2008 (2008-03-13) , pages 942-949, XP019624101, ISSN: 1619-7089**
- **A.Dimitrakopoulou-Strauss, L.G.Strauss: "PET Imaging of Prostate Cancer with 11 C-Acetate", The Journal of Nuclear Medicine, vol. 44 1 January 2003 (2003-01-01), 1 April 2003 (2003-04-01), pages 556-558, XP055229802, Retrieved from the Internet: URL:http://jnm.snmjournals.org/content/44/4/556.full.pdf#page=1&view=FitH [retrieved on 2015-11-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- TARUN SINGHAL ET AL: "11C-l-Methionine Positron Emission Tomography in the Clinical Management of Cerebral Gliomas", MOLECULAR IMAGING & BIOLOGY, vol. 10, no. 1, 24 October 2007 (2007-10-24), pages 1-18, XP055229805, Boston ISSN: 1536-1632, DOI: 10.1007/s11307-007-0115-2
- ANNA L. BARTELS ET AL: "Neuroinflammation in the pathophysiology of Parkinson's disease: Evidence from animal models to human in vivo studies with [ 11 C]-PK11195 PET", MOVEMENT DISORDERS., vol. 22, no. 13, 15 October 2007 (2007-10-15), pages 1852-1856, XP055229812, US ISSN: 0885-3185, DOI: 10.1002/mds.21552
- MILLER PHILIP W ET AL: "Synthesis of 11C, 18F, 15O, and 13N radiolabels for positron emission tomography", ANGEWANDTE CHEMIE INTERNATIONAL EDITION, WILEY - V C H VERLAG GMBH & CO. KGAA, DE, vol. 47, no. 47, 1 January 2008 (2008-01-01), pages 8998-9033, XP002609759, ISSN: 1433-7851, DOI: 10.1002/ANIE.200800222
- POLITIS M ET AL: "Evidence of dopamine dysfunction in the hypothalamus of patients with Parkinson's disease: An in vivo<11>C-raclopride PET study", EXPERIMENTAL NEUROLOGY, ACADEMIC PRESS, NEW YORK, NY, US, vol. 214, no. 1, 1 November 2008 (2008-11-01), pages 112-116, XP025562708, ISSN: 0014-4886, DOI: 10.1016/J.EXPNEUROL.2008.07.021 [retrieved on 2008-08-06]
- ROBERTSON, R. ET AL.: 'Optical imaging of Cerenkov light generation from positron-emitting radiotracers' PHYS MED BIOL. vol. 54, no. 16, 21 August 2009, pages N355 - 365, XP008156212

## Description

[Technical Field]

The present disclosure relates to a contrast agent for optical imaging, a use thereof and an apparatus using the same, and more particularly, to a contrast agent allowing for optical imaging even without having a fluorophore or a luminophore, a use thereof and an apparatus using the same.

[Background Art]

With the recent rapid development, various molecular imaging techniques allow to observe many in vivo processes, which was impossible in the past. Especially, nuclear imaging using radioisotopes is widely used in basic biological studies using small animals, preclinical researches and clinical applications, based on the advantages of high sensitivity, good tissue penetration and excellent quantification. The radionuclides may be categorized into five groups depending on their decay modes. They are: $\alpha$ decay, $\beta^-$ decay, $\beta^+$ decay, electron capture and isomeric transition. Among them, $\beta^+$-decaying nuclides ($^{18}F$, $^{11}C$, $^{13}N$, $^{15}O$, $^{64}Cu$, $^{124}I$ and $^{68}Ga$), which emit positrons during radioactive decay, are used to construct positron emission tomography (PET) images. The nuclides that emit gamma rays with appropriate energy during isomeric transition may be used for a gamma camera or single photon emission computed tomography (SPECT). $^{99m}Tc$ is the representative example. In $\beta^-$ decay, an electron is emited during the decay process. $^{3}H$, $^{14}C$, $^{35}S$, etc., which are widely utilized in radioisotope experiments in biological researches, belong to this category. Also, those widely used in radiation therapy using the emitted electrons, such as $^{32}P$, $^{131}I$, $^{89}Sr$, $^{90}Y$, $^{153}Sm$, $^{177}Lu$ and $^{186}Re$, belong to this category. The nuclides that decay by electron capture include $^{111}In$, $^{123}I$, $^{125}I$, $^{201}Tl$ and $^{67}Ga$. They may be used for gamma camera imaging since gamma ray is also emitted during the decay. As for the nuclides that undergo $\alpha$ decay, $^{211}At$ finds its application in radiation therapy.

A variety of radiopharmaceuticals have already been developed for use in gamma cameras and SPECT, whereby images are acquired based on gamma radiation, PET, wherein positron-emitting nuclides are used, or the like. They are widely used for accurate diagnosis of various diseases including cancer. Besides, therapeutic radiopharmaceuticals using various $\beta^-$- or $\alpha$-emitting radionuclides are gradually extending their applications. However, unlike optical imaging, nuclear imaging is not easily applicable in basic pre-screening stages since it requires expensive instruments such as SPECT or PET and expert operators. Furthermore, nuclear imaging has lower temporal resolution and requires more time to detect high-energy gamma rays and collect data for constructing images, as compared to the optical imaging.

In contrast to the nuclear imaging, optical imaging such as fluorescence and bioluminescence imaging is easy to handle and the instrumentation is much less expensive than PET or SPECT. Therefore, many basic and biomedical studies employ optical imaging to visualize molecular processes in living organisms. The optical imaging provides high sensitivity and excellent temporal resolution. However, quantification based on optical signals is not accurate and has limited applications in clinics due to the absorption and scattering of light in the body and low tissue penetration resulting therefrom.

Accordingly, there have been various attempts to combine nuclear imaging and optical imaging having their strong and weak points, and hybrid type imaging probes have been developed using nanoparticles such as quantum dots and near-IR fluorophores. However, in order to obtain both nuclear and optical images using one contrast agent, a hybrid type probe prepared by conjugating a radionuclide for nuclear imaging with a fluorescent or bioluminescent probe for optical imaging is needed. Especially, whereas nuclear imaging requires only one radioisotope to be introduced, optical imaging requires that a fluorophore or a luminophore (e.g., luciferase) be attached to the radioisotope. However, since most fluorophores or luminophores are bulky metal complexes, aromatic organic compounds or nanoparticles such as quantum dots, they change the physicochemical properties of the target molecules in many cases and, thus, their use in living organisms is extremely restricted. Although Korean Patent Publication No. 2007-0029030 discloses nanoparticles coated with a silica shell to confer both optical and magnetic properties, introduction of an organic fluorophore is still needed to exhibit optical properties.

From Robertson, R. et al., Phys Med Biol., 2009, vol. 54, no. 16, pages N355-365 it is known that positron-emitting radionuclides used in biomedical imaging have sufficient energy to result in Cerenkov radiation that can be observed with sensitive optical imaging equipment.

**[Disclosure]**

[Technical Problem]

The present disclosure is directed to providing a contrast agent including a radionuclide, which allows optical imaging without a fluorophore or a luminophore.

The present disclosure is also directed to providing a contrast agent including a radionuclide and a fluorophore, which allows optical imaging without energy input from outside by absorbing the energy emitted from the radionuclide and emitting light through the fluorophore, leading to stronger emission.

The present disclosure is also directed to providing a method for acquiring optical images of a living organism in a noninvasive manner in real time, by injecting a contrast agent including a radionuclide into the living organism.

The present disclosure is also directed to providing a contrast agent for trimodality imaging that can be prepared easier without having to attach a fluorophore and provides excellent resolution with superior tissue penetration, as compared to the existing contrast agents for trimodality imaging.

The present disclosure is also directed to providing various apparatuses for acquiring optical images, capable of converting the energy of a charged particle emitted from a contrast agent for optical imaging into light and, thereby, improving resolution of optical imaging.

The present invention is directed to a radionuclide detection apparatus for detecting the radionuclide labeled at a subject that can be subjected to HPLC and exists in a fluid.

[Technical Solution]

In one general aspect, the present disclosure provides a contrast agent for optical imaging, including a radionuclide which emits a charged particle having energy with a threshold T satisfying Equation 1 during radioactive decay:

[Equation 1]

$$T\ (\text{keV}) = 511\ [1 / (1 - 1/n^2)^{1/2} - 1]$$

where n is the refractive index of a medium.

In another general aspect, the present disclosure provides a contrast agent for optical imaging, including: a radionuclide which emits a charged particle having energy with a threshold T satisfying Equation 1 during radioactive decay; and a fluorophore, wherein the energy emitted from the radionuclide is accumulated in the fluorophore and light is emitted from the fluorophore:

[Equation 1]

$$T\ (\text{keV}) = 511\ [1 / (1 - 1/n^2)^{1/2} - 1]$$

where n is the refractive index of a medium.

According to a specfic embodiment of the present disclosure, the fluorophore may be one or more selected from a group consisting of a quantum dot nanoparticle, Cy3.5, Cy5, Cy5.5, Cy7, indocyanine green (ICG), Cypate, ITCC, NIR820, NIR2, IRDye78, IRDye80, IRDye82, Cresy Violet, Nile Blue, Oxazine 750, Rhodamine800 and Texas red.

In another general aspect, the present disclosure provides a method for acquiring optical images, including injecting the afore-described contrast agent for optical imaging into a living organism.

In another general aspect, the present disclosure provides a contrast agent for trimodality (optical/PET/MR) imaging, including a superparamagnetic nanoparticle labeled with a radionuclide which emits a charged particle having energy with a threshold T satisfying Equation 1 during radioactive decay:

[Equation 1]

$$T\ (\text{keV}) = 511\ [1 / (1 - 1/n^2)^{1/2} - 1]$$

where n is the refractive index of a medium.

In another general aspect, the present disclosure provides an apparatus for acquiring optical images by detecting light emitted from a contrast agent for optical imaging, including: a chamber accommodating a subject containing the contrast agent for optical imaging; a conversion means provided in the chamber and converting the energy of a charged particle emitted from the contrast agent for optical imaging into light; and a light detection means detecting the light converted by the conversion means.

The present invention provides a radionuclide detection apparatus for detecting the radionuclide labeled at a subject that can be subjected to HPLC and exists in a fluid flowing in a tube. It comprises a chamber through which the

tube penetrates, a conversion means provided in the chamber and converting the energy of a charged particle emitted from the radionuclide into Cerenkov light and a light detection means detecting the light converted by the conversion means. The tube has a diameter of 0.1-10 mm, has a light transmitting portion made of any transparent material allowing penetration of light and conversion of the energy of the charged particle into light by the conversion means, and is formed at a position facing the conversion means. The conversion means is made from a material having refractive index of at least 1.33 through which material light can pass, wherein the conversion means converts the energy of the charged particle passing through the light transmitting portion. The radionuclide emits a charged particle having energy with a threshold T satisfying Equation 1 during radioactive decay:

[Equation 1]

$$T\ (keV) = 511\ [1 / (1 - 1/n^2)^{1/2} - 1],$$

where n is refractive index of a medium.

The terms used in the specification will be described briefly.

Unless specificed otherwise, a "contrast agent for optical imaging" refers to a chemical or biological substance which allows, after being injected into a subject, acquisition of optical images by detecting fluorescent or luminescent light outside the subject.

A "multimodality contrast agent" refers to a contrast agent that can be used for different imaging modes, such as a contrast agent that can be used for both nuclear imaging (e.g., PET) and optical imaging or one that can be used for nuclear imaging, optical imaging and magnetic resonance (MR) imaging.

"Autoluminescence" refers to emission of light without energy input from outside.

An "optical/nuclear bimodal imaging contrast agent" refers to a contrast agent that can be used for both nuclear imaging (e.g., PET) and optical imaging.

To perform treatment and diagnosis at the same time means that radiation therapy and monitoring of, e.g., the effect of the radiation therapy by means of noninvasive nuclear or optical imaging are carried out using a single radiopharmaceutical.

[Advantageous Effects]

The contrast agent for optical imaging of the present disclosure does not require a fluorophore or a luminophore since it uses a radionuclide that emits light by converting the energy of a charged particle emitted during radioactive decay into light energy. Thus, optical images can be acquired while minimizing the change of physicochemical properties of the target molecules. Furthermore, when a fluorophore is attached, light emission may be enhanced without energy input from outside since light is emitted from the fluorophore. Accordingly, it may be widely used to acquire optical images in various applications including clinical studies. Especially, it is very useful in acquiring optical images of a living organism in real time, in a noninvasive manner.

When a superparamagnetic nanoparticle is labeled with a radionuclide satisfying Equation 1, an optical/PET/MR trimodal imaging contrast agent may be prepared simply and in high yield, without attachment of a fluorophore needed for conventional optical imaging.

In addition, the contrast agent for optical imaging of the present disclosure is applicable to commercially available optical imaging instruments to acquire optical images without any modification. The optical imaging using the radionuclide allows noninvasive imaging studies using numerous radiopharmaceuticals developed thus far and less expensive optical imaging instruments without further pretreatment processes, which were possible only with expensive instruments.

And, the apparatus for acquiring optical images of the present disclosure provides better resolution since it includes a conversion means converting the energy of a charged particle emitted from the contrast agent for optical imaging of the present disclosure into light. It also allows optical imaging using the same labeling method as that of nuclear imaging. Although it was difficult to detect the nuclides emitting little or no gamma rays (e.g., $\beta^-$-emitting nuclides such as $^{32}P$) using a radio-TLC scanner, an effect smiliar to that of radio-TLC scanning may be attained with the optical imaging.

[Description of Drawings]

The above and other objects, features and advantages of the present disclosure will become apparent from the following description of certain exemplary comparative and clarifying embodiments of the disclosure and one embodiment of the invention which embodiments are given in conjunction with the accompanying drawings, in which:

Fig. 1 schematically illustrates an apparatus for acquiring optical images according to a specific embodiment of the present disclosure.

Fig. 2 schematically illustrates an apparatus for acquiring optical images for medical use according to a specific embodiment of the present disclosure.

Fig. 3 schematically illustrates an endoscope according to a specific embodiment of the present disclosure.

Fig. 4 schematically illustrates a radionuclide detection apparatus for medical use according to a specific embodiment of the present invention.

Fig. 5a shows a luminescence image of a BALB/c mouse at 1 hour post-injection of [$^{18}$F]FDG, and Fig. 5b shows a luminescence image of the mouse (C3H/HeN) at 40 minutes post-injection of [$^{124}$I]NaI.

Fig. 6a shows a luminescence image of a 96-well plate containing 300 $\mu$L of an aqueous solution of $^{124}$I, $^{18}$F, $^{68}$Ga, $^{131}$I and $^{99m}$Tc at various concentrations, Fig. 6b shows a plot of luminescence intensity versus radioactivity of different radionuclides, and Fig. 6c shows intensity of the luminescence signal measured by an in vitro luminometer as a function of activity concentrations.

Fig. 7 shows a luminescence image (a) and a microPET image (b) of a nude mouse bearing NIH3T6.7 tumor cells at 2 days post-injection of $^{124}$I-labeled Herceptin (3.29 MBq). The tumor sites are indicated by red arrow heads (scan time: luminesence = 1 min, microPET = 20 min). Also shown are ex vivo luminesence (c), optical (d) and microPET (e) images of organs excised immediately after in vivo imaging (Ts: thymus, Td: thyroid, Hr: heart, Lg: lung, Lv: liver, St: stomach, Ms: muscle, Kd: kidneys, Sp: spleen, T(s): tumor in shoulder, T(f): tumor in flank. scan time: optical = 3 min, microPET = 20 min).

Fig. 8 shows a synthetic scheme of tyramine-conjugated poly(TMSMA-r-PEGMA-r-NAS) and a schematic of $^{124}$I-labeled TCL-SPION (a), UV analysis results of the tyramine-conjugated polymer and poly(TMSMA-r-PEGMA-r-NAS) (b), and a mean hydrodynamic diameter of the tyramine-conjugated TCL-SPION determined by electrophoretic light scattering (c).

Fig. 9 shows a spin-spin relaxation rate (R2) of a tyramine-conjugated TCL-SPION versus iron concentration.

Fig. 10 shows luminescence (a), microPET (b) and T2-weighted MR (c) images of a mixture of $^{124}$I and TCL-SPION at different concentrations. The most concentrated solution [762 $\mu$Ci/mL (3.0 ng/mL) $^{124}$I + 205 $\mu$g/mL TCL-SPION] was serially diluted by 1/3. Also shown are luminescence (d) and microPET (e) images of a Derenzo phantom containing 32 $\mu$Ci/mL $^{124}$I solution. A series of different concentrations of $^{124}$I (10 $\mu$L each) was injected into the backs of Sprague-Dawley rats at depths of 4 mm (f, g) and 7 mm (h).

Fig. 11 shows optical (a), microPET (b) and MR (c) images of $^{124}$I-labeled SPIONs injected into the front paws of a BALB/c mouse bearing 4T1 tumor cells implanted on its shoulder (tumor: yellow arrow; sentinel lymph nodes: red dotted circles; injection sites: "I"; bladder: red arrow; fiducial markers: white arrow heads). Also shown are ex vivo luminescence (top) and microPET (bottom) images (d) of the dissected lymph nodes and a schematic (e) of a tumor metastasis model and injection route of radiolabeled nanoparticles.

Fig. 12 shows a PET/MR fusion image.

Fig. 13 shows a result of histological examination of tumor and lymph nodes. a-c) show H&E staining results of tumor and brachial lymph nodes. d-f) CK8/18-positive cells are epithelial-derived tumor cells. g-i) TCL-SPIONs existing in the tissue appear bright blue through staining with Prussian blue (Left LN: left brachial lymph node; Right LN: right brachial lymph node, PB: Prussian blue staining, scale bar = 50 $\mu$m).

Fig. 14 shows photographic (A) and luminescence (B) images of a plant (arabidopsis) at 10 minutes after immersion in water (left, control) or in a [$^{32}$P]phosphoric acid solution. Radiation from selected leaves (red ovals in (A)) with time is shown in (C).

Fig. 15 shows luminescence images of sequentially diluted [$^{99m}$Tc]$TcO_4^-$ solutions (300 $\mu$L/well).

Fig. 16 shows a plot of luminescence intensity as a function of activity concentration for [$^{18}$F]FDG and [$^{18}$F]KF.

Fig. 17 shows luminescence emission spectra of a $^{124}$I solution (blue line), a mixed solution of $^{124}$I and quantum dots (red line), and an aqueous solution of quantum dots only (black line) as a control.

Fig. 18 shows a luminescence image of a C18 TLC plate spotted with the $^{131}$I-labeled compounds (A, scan time: 1 min), a radio-TLC chromatogram (B, scan time: 2 min), and a relative percentage of ROI of luminescence imaging and radio-TLC (C).

Fig. 19 a) shows lines drawn first on white paper with a pencil and some lines drawn over the lines with a $^{32}$P solution (1 mCi/100 $\mu$L in water). b) shows the words "CERENKOV RADIATION" written with a $^{32}$P solution (920 $\mu$C/100 $\mu$L). The luminescence images of the lines were obtained at 1 minute before (left) and after (right) covering the paper with a 1.2 mm-thick transparent glass plate.

[Detailed Description of Main Elements]

100: apparatus for acquiring optical images
110: light detection means
111: CCD 120: lens or filter
130: conversion means 140: supporting means

[Best Mode]

Hereinafter, the comparative and clarifying embodiments of the present disclosure and the embodiment of the invention will be described in detail with reference to accompanying drawings.

As described above, there have been various attempts to combine nuclear imaging and optical imaging having their strong and weak points, and hybrid type imaging probes have been developed using nanoparticles such as quantum dots and near-IR fluorophores. However, in order to obtain both nuclear and optical images using one contrast agent, a hybrid type probe prepared by conjugating a radionuclide for nuclear imaging with a fluorescent or bioluminescent probe for optical imaging is needed. Especially, whereas the nuclear imaging requires only one radioisotope to be introduced to obtain nuclear images, the optical imaging requires that a fluorophore or a luminophore be attached to the radioisotope. However, since most fluorophores or luminophores are bulky metal complexes, aromatic organic compounds or nanoparticles such as quantum dots, they change the physicochemical properties of the target molecules in many cases and, thus, their use in living organisms is restricted.

The inventors of the present disclosure have confirmed that specific radionuclides can emit light even without a fluorophore or a luminophore and that optical images can be acquired by detecting the emitted light. Accordingly, when the specific radionuclides are used, for example, as a contrast agent, optical images can be acquired easily without having to attach a fluorophore. As a result, the optical images can be acquired in real time without changing the physicochemical properties of a substrate.

A contrast agent for optical imaging according to an embodiment of the present disclosure comprises a radionuclide which emits a charged particle having energy with a threshold T satisfying Equation 1 during radioactive decay:

[Equation 1]

$$T\ (\text{keV}) = 511\ [1 / (1 - 1/n^2)^{1/2} - 1]$$

where n is the refractive index of a medium.

Equation 1 is an equation about the threshold for the emission of Cerenkov radiation. Autoluminescence can occur from the radionuclide when it emits a charged particle having energy with a threshold T satisfying Equation 1.

Fig. 5a shows a luminescence image of a BALB/c mouse bearing luciferase-transfected 4T1 tumour cells taken in luminescence mode at 1 hour after injecting the PET tracer [$^{18}$F]FDG ($^{18}$F-analog of glucose, 409 $\mu$Ci). An intense signal was detected in the brain and brown fat area on the shoulder. The injection route of [$^{18}$F]FDG (tail) was also clearly visualized. Because there is no external light source in the luminescence imaging mode and no luciferin had been injected before, the light signal is believed to have originated from the injected radiotracer. The luminescence image is also well matched with the expected biodistribution pattern of high [$^{18}$F]FDG uptake in the brain and brown fat. A different radionuclide, [$^{124}$I]NaI ion (500 $\mu$Ci), was injected intraperitoneally into a normal mouse (C3H/HeN) and the luminescence image was measured in an attempt to detect radionuclides by luminescence imaging (Fig. 5b). As expected, the image clearly showed high uptake in the thyroid and bladder region, which also suggests that the light signals originated from the injected radioiodines.

The origin and properties of the luminescence light were characterized systematically using five different radioisotope samples at various activity concentrations and taking luminescence images (Fig. 6). The high dependence of the luminescence intensity on radioisotope species was clearly observed. While the positron emitter $^{68}$Ga showed the most intense light signal, the $\gamma$-ray emitter, $^{99m}$Tc, did not show any detectable light signal at similar activity concentrations (Fig. 6a). The other two positron emitters, $^{124}$I and $^{18}$F, showed the second and third strongest luminescence signals, respectively, and although the luminescence signal of the $\beta$- emitter, $^{131}$I, was not as strong as the other three positron emitters, it also showed a sufficiently detectable light signal. The strongest light emitter, $^{68}$Ga, could be detected clearly by luminescence imaging up to 0.5 $\mu$Ci/mL in 300 $\mu$L of water, but aqueous $^{99m}$Tc solution of the same volume emitted only very faint light at 2.9 $\times$ 10$^3$ $\mu$Ci/mL. The intensity of the luminescence signals showed a linear correlation with the level of radioactivity (Fig. 6b). All the luminescence signals could be blocked completely by covering the well with opaque black paper, which suggests that the signal originates from the light emitted from inside the well, not by any direct interference between the charge-coupled device (CCD) detector and the high energetic radiation produced during decay.

The relationship between the luminescence signal and decay mode of each radionuclide was examined further and the intensity of the luminescence signal depending on radioactivity was quantified by in vitro luminometric assay. Eight different radionuclide samples, which decay in various decay modes, were prepared at consecutively diluted concentrations (Fig. 6c). A similar relationship between the luminescence intensity and radioisotope species was observed. In general, all radionuclides showed an increase in luminescence intensity with increasing activity. However, three isotopes, $^{111}$In, $^{99m}$Tc and $^{35}$S, showed a much slower increase in light signal compared to the other five nuclides, $^{32}$P, $^{124}$I, $^{18}$F, $^{131}$I and $^{64}$Cu. In particular, $^{35}$S did not show any noticeable increase in luminescence even at the highest activity. There was no significant difference in light intensity depending on chemical forms ([$^{18}$F]FDG vs. [$^{18}$F]KF, Fig. 16).

A close examination of the physical properties of the selected radionuclides revealed a good correlation between the luminescence intensity and the energy of particles emitted during decay, such as positrons and electrons, rather than the decay mode, Q-value, and energy of $\gamma$-rays emitted from radionuclides (Table 1).

[Table 1] Physical properties[a] of various radionuclides and relative luminescence intensity

| Nuclides (half-life) | $^{68}$Ga (68 min) | 32p (14.3 d) | $^{124}$I (4.2 d) | $^{18}$F (110 min) | $^{131}$I (8.0 d) | $^{64}$Cu (12.7 h) | $^{111}$In (2.8 d) | $^{99m}$Tc (6.0 h) | $^{35}$S (87.5 d) |
|---|---|---|---|---|---|---|---|---|---|
| Decay mode (%) | $\beta^+$ (89) EC (11) | $\beta^-$ (100) | $\beta^+$ (23) EC (77) | $\beta^+$ (97) EC (3) | $\beta^-$ (100) | $\beta^+$ (17) $\beta^-$ (39) | EC (100) | IT (100) $\beta$-(3.7x10$^{-5}$) | $\beta^-$ (100) |
| $E_{\beta+\ mean}$, keV (%) | 829 (89) | | 819 (23) | 250 (97) | | 278 (17) | | | |
| $E_{\beta-\ mean}$, keV (%) | | 695 (100) | | | 182 (100) | 190 (39) | | 114 (3.7x10-$^{5}$) | 49 (100) |
| $E_{ce\ mean}$, keV (%) | | | | | | | 176 (16) | 14 (110) | |
| Relative intensity[b] (IVIS200)[c] | (24.2) | 25.9 | 5.1 (6.2) | 2.0 (1.7) | 1 (1) | 0.8 | 0.1 | 0.04 | 0.02 |

[a] Physical properties of the radionuclides were adapted from MIRD-07. [b] Relative luminescence intensity was calculated from in vitro luminometer data with respect to the luminescence signal of $^{131}$I set to 1. [c] Relative intensity was calculated from the IVIS 200 data. EC: electron capture, **IT:** isomeric transition, ce: conversion electron.

Among the five nuclides emitting strong to moderate luminescence signals, three nuclides, $^{68}$Ga, $^{124}$I and $^{18}$F, decayed via $\beta^+$ decay and emitted positrons with $\beta^+$ mean energy ($E_{\beta+}$ mean) of 829, 819 and 250 keV, respectively. On the other hand, $^{32}$P and $^{131}$I decayed via one hundred percent $\beta^-$ decay and also emitted $\beta^-$ particles with $E_{\beta-\ mean}$ of 695 and 182 keV, respectively. $^{64}$Cu followed both $\beta^+$ and $\beta^-$ decay modes and emitted both high energy positron ($E_{\beta+\ mean}$ 278 keV) and electron ($E_{\beta-\ mean}$ 190 keV). However, three nuclides, $^{111}$In, $^{99m}$Tc and $^{35}$S, with weak luminescence signal emitted only low energetic $\beta^-$ particles or very small quantity of $\beta^-$ particles.

To conclude, luminescence occurs during radioactive decay of some radionuclides and optical images can be acquired only with the radionuclides without additional fluorophore or luminophore.

The good correlation between the luminescence intensity and energy of charged particle emitted during decay is reminiscent of Cerenkov radiation. Cerenkov radiation is the light emitted when a charged particle travels with a velocity greater than that of light in a given medium. When a charged particle traveling with a velocity greater than that of light in a medium loses its kinetic energy and slows down to the speed of light, the decreased kinetic energy is turned into light energy. A typical example of Cerenkov radiation is the bluish glow observed around an operating nuclear reactor core. Until now, the applications of Cerenkov radiation have been concerned primarily with cosmic-ray and high-energy physics measurements. Some high energy $\beta$-emitting nuclides, such as $^{32}$P, were measured using Cerenkov light, which is commonly known as 'Cerenkov counting'. The threshold for the emission of Cerenkov radiation can be calculated by Equation 1.

[Equation 1]

$$T\ (keV) = 511\ [1 / (1 - 1/n^2)^{1/2} - 1]$$

where T is the threshold for the emission of Cerenkov radiation, and n is the refractive index of the medium.

Because water has a refractive index of 1.33, the minimum energy for the emission of Cerenkov radiation is 262 keV when a contrast agent comprising the radionuclide of the present disclosure is injected into a living organism. Therefore, theoretically any radionuclide that emits a positron or β-particle with energy > 262 keV during decay can emit Cerenkov radiation and be detected using a high-sensitivity luminescence detector. This explains why high energy electron- and positron-emitting nuclides, such as $^{68}$Ga, $^{32}$P, $^{124}$I, $^{18}$F, $^{131}$I and $^{64}$Cu, emit strong luminescence light while $^{111}$In, $^{99m}$Tc and $^{35}$S emit very weak light signals.

The charged particle emitted during the decay of a radionuclide has a varying energy distribution. To take $^{18}$F as an example, the emitted charged particle has a mean energy of 250 keV. Since the maximum emission energy is 634 keV, the emitted charged particle has a conituous energy distribution between 0 and 634 keV. From the requirement of Equation 1, the minimum energy required for radiation in air (refractive index = 1.0003) is 20355 keV. Accordingly, light is not emitted for the radionuclide, since the maximum energy is only 634 keV. However, if the medium is water (refractive index = 1.33), the minimum energy required for radiation is smaller than in air, with 262 keV. Thus, among the emitted charged particles, one having an energy of 262 keV or higher can emit light. In other words, when a charged particle traveling with a speed greater than that of light loses its kinetic energy and slows down to the speed of light, the decreased kinetic energy is turned into light energy. If the medium is glass (refractive index = 1.52) with a refractive index higher than that of water, a charged particle having an energy 168 keV or higher can emit light. In this example, although luminescence occurs both in water or glass, glass having a higher refractive index requires less energy for radiation than water having a lower refractive index. As a result, the energy emitted from charged particles with a broader energy distribution can be converted into light, and thus light emission is enhanced.

Specifically, as seen from Figs. 18 and 19, more distinct optical images could be obtained when a medium having a higher refractive index (e.g., refractive index of glass = 1.52) than a subject including the radionuclide was used. Specifically, in Fig. 19, when the medium was air with a low refractive index (left), the image was unclear since the energy reuiqred for Cerenkov radiation is high. In contrast, when the medium was glass with a high refractive index (right), a clear image could be attained since a lower energy is required.

However, the medium is not limited to glass. Depending on the type and state of the subject, air (refractive index = 1.0003), water (refractive index = 1.33) or other materials with high refractive indices (e.g., glass, high refreactive plastic, etc.) may be used as a medium to enhance the intensity of Cerenkov emission from the radionuclide. By using a material with a high refractive index as the medium, the minimum energy required for the emission of Cerenkov light can be reduced and, hence, the light emission can be improved. Moreover, by injecting a substance with a high refractive index into the subject, more distinct optical images can be obtained since the light emission is enhanced.

The charged particle emitted from the radionuclide may be electron, positron or α particle depending on the mode of radioactive decay.

The intensity of a photon emitted by Cerenkov radiation is proportional to $1/\lambda^2$ and increases as the energy of the charged particle increases beyond the Cerenkov threshold. This explains further why the relative intensity of $^{68}$Ga and $^{32}$P is higher than that of $^{124}$I, $^{18}$F, $^{131}$I and $^{64}$Cu. Although the mean positron energy of $^{68}$Ga is similar to $^{124}$I (829 vs. 819 keV), the relative intensity of $^{68}$Ga is more than 4 times higher than that of $^{124}$I because the branching ratio to the positron decay of $^{68}$Ga is approximately 4 times larger than that of $^{124}$I (89 vs. 23%). The radionuclide satisfying Equation 1 is likely to be a radionuclide following $\beta^+$, $\beta^-$ or electron capture decay. In this regard, the radionuclide that can be employed in the present disclosure may be a radionuclide that decays via $\beta^+$ decay such as $^{10}$C, $^{11}$C, $^{13}$O, $^{14}$O, $^{15}$O, $^{12}$N, $^{13}$N, $^{15}$F, $^{17}$F, $^{18}$F, $^{32}$Cl, $^{33}$Cl, $^{34}$Cl, $^{43}$Sc, $^{44}$Sc, $^{45}$Ti, $^{51}$Mn, $^{52}$Mn, $^{52}$Fe, $^{53}$Fe, $^{55}$Co, $^{56}$Co, $^{58}$Co, $^{61}$Cu, $^{62}$Cu, $^{62}$Zn, $^{63}$Zn, $^{64}$Cu, $^{65}$Zn, $^{66}$Ga, $^{66}$Ge, $^{67}$Ge, $^{68}$Ga, $^{69}$Ge, $^{69}$As, $^{70}$As, $^{70}$Se, $^{71}$Se, $^{71}$As, $^{72}$As $^{73}$Se, $^{74}$Kr, $^{74}$Br, $^{75}$Br, $^{76}$Br, $^{77}$Br, $^{77}$Kr, $^{78}$Br, $^{78}$Rb, $^{79}$Rb, $^{79}$Kr, $^{81}$Rb, $^{82}$Rb, $^{84}$Rb, $^{84}$Zr, $^{85}$Y, $^{86}$Y, $^{87}$Y, $^{87}$Zr, $^{88}$Y, $^{89}$Zr, $^{92}$Tc, $^{93}$Tc, $^{94}$Tc, $^{95}$Tc, $^{95}$Ru, $^{95}$Rh, $^{96}$Rh, $^{97}$Rh, $^{98}$Rh, $^{99}$Rh, $^{100}$Rh, $^{101}$Ag, $^{102}$Ag, $^{102}$Rh, $^{103}$Ag, $^{104}$Ag, $^{105}$Ag, $^{106}$Ag, $^{108}$In, $^{109}$In, $^{110}$In, $^{115}$Sb, $^{116}$Sb, $^{117}$Sb, $^{115}$Te, $^{116}$Te, $^{117}$Te, $^{117}$I, $^{118}$I, $^{118}$Xe, $^{119}$Xe, $^{119}$I, $^{119}$Te, $^{120}$I, $^{120}$Xe, $^{121}$Xe, $^{121}$I, $^{122}$I, $^{123}$Xe, $^{124}$I, $^{126}$I, $^{128}$I, $^{129}$La, $^{130}$La, $^{131}$La, $^{132}$La, $^{133}$La, $^{135}$La, $^{136}$La, $^{140}$Sm, $^{141}$Sm, $^{142}$Sm, $^{144}$Gd, $^{145}$Gd, $^{145}$Eu, $^{146}$Gd, $^{146}$Eu, $^{147}$Eu, $^{147}$Gd, $^{148}$Eu, $^{150}$Eu, $^{190}$Au, $^{191}$Au, $^{192}$Au, $^{193}$Au, $^{193}$Tl, $^{194}$Tl, $^{194}$Au, $^{195}$Tl, $^{196}$Tl, $^{197}$Tl, $^{198}$Tl, $^{200}$Tl, $^{200}$Bi, $^{202}$Bi, $^{203}$Bi, $^{205}$Bi or $^{206}$Bi, a radionuclide that decays via $\beta^-$ decay such as $^{3}$H, $^{14}$C, $^{35}$S, $^{32}$P, $^{131}$I, $^{59}$Fe, $^{60}$Co, $^{67}$Cu, $^{89}$Sr, $^{90}$Sr, $^{90}$Y, $^{99}$Mo, $^{133}$Xe, $^{137}$Cs, $^{153}$Sm, $^{177}$Lu or $^{186}$Re, or a radionuclide that decays via electron capture such as $^{111}$In, $^{123}$I, $^{125}$I, $^{201}$Tl, $^{67}$Ga, $^{51}$Cr, $^{57}$Co, $^{58}$Co, $^{62}$Zn or $^{82}$Sr. Most specifically, it may be $^{18}$F, $^{11}$C, $^{13}$N, $^{15}$O, $^{60}$Cu, $^{64}$Cu, $^{67}$Cu, $^{124}$I, $^{68}$Ga, $^{52}$Fe, $^{58}$Co, $^{3}$H, $^{14}$C, $^{35}$S, $^{32}$P, $^{131}$I, $^{59}$Fe, $^{60}$Co, $^{89}$Sr, $^{90}$Sr, $^{90}$Y, $^{99}$Mo, $^{133}$Xe, $^{137}$Cs, $^{153}$Sm, $^{177}$Lu, $^{186}$Re $^{123}$I, $^{125}$I, $^{201}$Tl or $^{67}$Ga, but is not limited thereto. Since other radionuclides that do not decay via $\beta^+$, $\beta^-$ or electron capture can also emit light, they may also be used as the radionuclide of the present disclosure if Equation 1 is satisfied.

Thus, it is thought that Cerenkov radiation is the main contribution of the luminescence occurring during radioactive decay of the radionuclide according to the present disclosure, and it may be utilized in a contrast agent and for optical imaging without having to attach a fluorophore or a luminophore. Specifically, NIH3T6.7 tumours implanted in a nude mouse were clearly visualized by $^{124}I$-labeled Herceptin (trastuzumab) antibody in luminescence imaging (Fig. 7, A). Whilst Herceptin was conjugated with relatively bulky fluorophores, such as Cy 5.5, RhodG and quantum dots, for optical imaging in previous studies, the Herceptin in the present study was simply radioiodinated with $^{124}I$ (89 $\mu$Ci) using a well-established Iodo-Beads method, in which an oxidized $I^+$ ion reacts with the tyrosine residue of the antibody. Because $^{124}I$ is a positron emitter, the $^{124}I$-labeled Herceptin can also be imaged using a microPET scanner (Fig. 7, B). Two tumour sites were also clearly visualized in the microPET images. Uptake in the internal organ was also visualized, which was not observed in the optical images, presumably due to the absorption and scattering of the light signal by the tissue (Fig. 7, C-E).

The application of Cerenkov imaging can be extended easily to multimodality imaging. A single imaging probe radiolabeled with an appropriate radioisotope can be used as an optical and nuclear dual imaging probe on account of the intrinsic dual imaging potential of the radionuclide emitting high energetic particles during decay.

In a specific embodiment, the present disclosure provides a contrast agent for trimodality (optical/PET/MR) imaging, comprising a superparamagnetic nanoparticle labeled with a radionuclide which emits a charged particle having energy with a threshold T satisfying Equation 1 during radioactive decay:

[Equation 1]

$$T\ (\text{keV}) = 511\ [1 / (1 - 1/n^2)^{1/2} - 1]$$

where n is the refractive index of a medium.

Multimodality imaging has been considered as an important diagnosis tool during the past decades in basic biomedical researches and clinical studies. Nevertheless, the currently available imaging modalities, including optical, X-ray, nuclear, magnetic resonance (MR) and ultrasound imaging, cannot provide all the information needed for elucidating the complicated biological events in living organisms. Each imaging modality has its own advantages and disadvantages in terms of sensitivity, temporal and spatial resolution, tissue penetration, quantification, instrument cost, availability, radiation exposure, functional information, and so forth. Therefore, the combination of two or more imaging modalities often provide the combined advantages of each while compensating for the weak points of each modality. Among the various multimodality imaging agents that have been developed in this context to date, optical/nuclear, optical/MR and nuclear/MR dual imaging probes have attracted special interest because of the high degree of complementarity among these three optical, nuclear and MR imaging modalities. Optical imaging techniques such as fluorescence and bioluminescence imaging can be obtained in short scanning time (< 1 min) with high sensitivity using relatively cheap imaging instruments. But, they suffer from a low tissue penetration problem, leading to poor quantification and limitation in use to imaging of small animals. Nuclear imaging such as positron emission tomography (PET) has the advantages of high sensitivity and accurate quantification, even for humans, but its spatial resolution is poor, being limited to the range of several millimeters. On the other hand, MR imaging (MRI) can provide excellent anatomical information in the sub-millimeter range, especially for soft tissue, but its sensitivity is far inferior to that of optical and nuclear imaging. Nuclear imaging and MRI both provide three-dimensional (3D) tomographic images for clinical use, but the high cost of imaging scanners has limited access by basic researchers.

Therefore, the development of an optical/PET/MR imaging probe will provide numerous benefits. Using a single hybrid probe, the biological events of interest in living organisms can be quickly screened with high sensitivity using an easily accessible optical imaging instrument, so that, subsequently, only the selected subjects are subjected to PET and MR imaging for further accurate visualization of the injected probe supported by detailed 3D anatomical information. In many cases, however, integrating individual imaging probes into a single hybrid system to achieve such multimodality imaging requires multi-step synthesis and purification processes, which often result in significant loss of optical, PET and MR signals. This explains why only a few examples of triple-modality imaging probes have been reported so far. Therefore, a facile method for the preparation of a triple-modality probe is needed.

The triple-modality imaging probe of the present disclosure can be constructed by conjugating an appropriate PET radionuclide that is able to generate Cerenkov light to a magnetic probe. Unlike the existing methods that combine three different individual probes to generate triple-modality images, the probe can be prepared by using only two different components: the PET and MR imaging agents.

In order to evaluate the feasibility of using Cerenkov light as a new optical imaging modality, the inventors selected a $^{124}I$ radioisotope among the many PET radionuclides due to its sufficiently long half-life (4.2 days) for probe preparation, and more importantly, its emission of high-energy positrons during its decay with a $\beta^+$ mean energy ($E_{\beta+\ \text{mean}}$) of 819 keV, leading to strong Cerenkov radiation. On the other hand, they selected a thermally cross-linked, superparamagnetic iron oxide nanoparticle (TCL-SPION) as an MRI contrast agent due to its high stability in vivo and excellent anti-biofouling property owing to the cross-linked polymer coating layers containing polyethylene glycol (PEG) on the magnetite core.

To evaluate the sensitivity of the Cerenkov luminescence imaging, a series of solutions (300 μL) containing $^{124}$I and TCL-SPION were imaged by optical, microPET and MR imaging (Fig. 10, a)-c)). One of the most noticeable features of Cerenkov imaging in comparison with microPET and MR imaging is the wide range of contrast in the luminescence images. A range of activity concentrations varying from 762 to 3.14 μCi/mL $^{124}$I was clearly visualized and, more importantly, the concentrations were distinguishable from each other. The sensitivity and contrast of Cerenkov imaging were notable, given the imaging time of optical imaging (1 min) versus that of microPET and MR imaging (20 min for both). In the MR images, the solutions containing up to 0.84 μg/mL TCL-SPION showed noticeable contrast compared to blank water, but consideration of the 3.0 ng mass of 762 μCi of $^{124}$I clearly indicated the much lower sensitivity of MRI compared to that of Cerenkov imaging and PET imaging. Furthermore, the three most concentrated solutions could not be differentiated from one another in the MRI.

The spatial resolution of Cerenkov imaging was studied using a Derenzo phantom having holes of different sizes filled with radioactive sources. A Derenzo phantom containing 32 μCi/mL $^{124}$I solution was imaged by luminescence and microPET (Fig. 10, d) and e)). Cerenkov imaging could clearly identify each source up to a resolution of 1.6 mm and some sources could be separated from other nearby sources with a resolution of 1.2 mm, while a maximum resolution of 2.4 mm was achieved in microPET imaging. This demonstrated the higher spatial resolution of optical imaging based on Cerenkov radiation compared to microPET imaging, at least for in vitro imaging. The major potential concern with using Cerenkov imaging as a tool for in vivo imaging is its anticipated low tissue penetration because Cerenkov radiation emits high-intensity photons at wavelengths in the blue light region, and its intensity decreases in proportion to $1/\lambda^2$. Cerenkov luminescence images of Sprague-Dawley rats injected with different amounts of $^{124}$I on the back muscle at depths of 4 mm and 7 mm are shown in Fig. 10, f)-h). When 10 μL of $^{124}$I solution was injected at a depth of 4 mm and imaging was performed for 1 minute, the lowest concentration of $^{124}$I solution detected was 0.3 μCi/μL (Fig. 10, f)). A longer scan time enabled detection of an even lower concentration of 0.1 μCi/μL of $^{124}$I, and each injection site was clearly differentiated at a separation of 1 cm (Fig. 10, g)). When the injection depth was increased to 7 mm, the minimum activity for detection was increased to 1 μCi/μL (10 μL, scan time = 1 min). The results of these tissue penetration experiments clearly demonstrated the successful application of Cerenkov imaging with small amounts of radioactivity for various in vivo imaging studies using small animals such as mice and rats.

The great potential of Cerenkov radiation as a new optical imaging modality and, hence, the intrinsic optical/PET dual-imaging property of some PET radionuclides provide a simpler method of preparing a hybrid optical/PET/MR imaging nanoprobe by conjugating an appropriate PET radionuclide ($^{124}$I) to an MRI probe (TCL-SPION). A schematic illustration for the preparation of the $^{124}$I-labeled TCL-SPION is shown in Fig. 8, a). In the first step, the poly(TMSMA-r-PEGMA-r-NAS) that was originally used as a coating material in the preparation of TCL-SPION was partially modified by tyramine (4-(2-aminoethyl)phenol) that can easily introduce an iodine at the ortho-position of the phenol ring. In the second step, $Fe_3O_4$ magnetite nanoparticles sized 4-11 nm (see the insert of Fig. 8, c)) were coated by the hydrolyzed form of the tyramine-conjugated polymer, and the polymer coating layers were subsequently cross-linked by heating to give tyramine-conjugated TCL-SPIONs. The content of tyramine in the tyramine-conjugated polymer was calculated by UV analysis to be 0.77 wt % (Fig. 8, b)). The hydrodynamic particle size of the tyramine-conjugated TCL-SPIONs was measured to be 39 ± 8 nm (Fig. 8, c)). The nanoparticles were dispersed well in aqueous solution and were stable up to six months without any aggregation. Their T2 relaxivity coefficient (r2), measured as 283.7 $mM^{-1}s^{-1}$, was higher than that of conventional iron oxide-based SPIONs (Feridex, 234.2 $mM^{-1}s^{-1}$) (Fig. 9).

The radiolabeling of the tyramine-conjugated TCL-SPIONs with $^{124}$I was easily accomplished using commercially available lodo-Beads (Pierce Biochemical Co., USA). Radioactive iodine offers the significant two advantages of well-established radioiodination and a high radiolabeling yield that can easily be achieved using one of several commercially available labeling procedures. In the treatment of [$^{124}$I]NaI with lodo-Beads, cationic iodine species (ICl) are formed and these reactive electrophiles form bonds with the phenol group at the ortho position (Fig. 8, a)). The radiolabeling yield was 79% and the radiochemical purity of the $^{124}$I-labeled TCL-SPIONs was 92% after centrifugal purification.

To examine the potential of the $^{124}$I-labeled TCL-SPIONs as a triple-modality imaging probe in vivo, the inventors performed sentinel lymph node imaging. Accurate imaging of the sentinel lymph node, especially the differentiation of a metastasized lymph node against tumor-free sentinel lymph nodes, is crucial, because many tumors spread out to other organs via the lymphatic vessels, and the surgical dissection area is determined based on the location of the metastasized sentinel lymph nodes. The tyramine-conjugated TCL-SPIONs have a hydrodynamic size of about 40 nm, which is included in the ideal size range for a lymph node imaging agent (5-50 nm), and its high in vivo stability and anti-biofouling properties further increase its suitability for lymph node imaging.

A tumor metastasis model was prepared by injecting the mouse breast cancer cell line 4T1 into the left shoulder of normal BALB/c mice. About ten days later, the $^{124}$I-labeled TCL-SPIONs (38 μg, 20 μCi per injection) were injected into both front paws of a 4T1 tumor-bearing BALB/c mouse. Luminescence, microPET and MR imaging were then

performed to see if the metastasized lymph node could be differentiated from a tumor-free sentinel lymph node (Fig. 11). All the three imaging modalities consistently showed a much lower uptake of the injected nanoparticles in the left brachial sentinel lymph node located close to the tumor site, presumably due to the metastasis of the 4T1 tumor cells into the closer lymph node and the destruction of the lymph node function. However, Cerenkov imaging was superior to the other imaging modalities in terms of its easy identification of the lymph nodes with minimal background and anatomical information supported by photographic images (Fig. 11, a)). The autofluorescence background is one of the shortcomings of the fluorescence imaging modality. However, as a type of luminescence imaging, Cerenkov imaging does not suffer from this annoying optical interference because it does not need any external light source as in the case of bioluminescence imaging. In addition to the injection sites and the lymph nodes, the signal was also seen in the bladder region, presumably due to the urinal excretion of the co-injected unpurified free radioiodine. Because the microPET image was also reconstructed based on the signal from $^{124}I$, its image was well matched with the Cerenkov imaging results. Even though the precise location of one strong signal around the right brachial lymph node was not clear in the PET images because of its poor anatomical information, the microPET imaging offered the advantage of accurate visualization of the activity distribution in the internal organs, thanks to its excellent tissue penetration nature (Fig. 11, b)). The strong dark spot could be unambiguously assigned to the brachial lymph node on the basis of the detailed tomographic anatomical information provided by MRI (Fig. 11, c)). The microPET-MR fusion image guided by fiducial markers revealed the perfect match of the strong blue spot in the microPET image with the intense dark spot in the MR image. The ex vivo optical and microPET images of the dissected lymph node were well matched to the in vivo imaging results (Fig. 11, d)). The immunostaining studies of the excised lymph nodes confirmed that the sentinel lymph node close to the implanted tumor was actually metastasized by the 4T1 tumor cells and had a lower uptake of iron-based nanoparticles.

Although the superparamagnetic iron oxide-based nanoparticle (TCL-SPION) was described as an example of the superparamagnetic nanoparticle that can be used in the present disclosure, other various known superparamagnetic nanoparticles may also be used. The superparamagnetic nanoparticle may be modified partly or wholly on its surface in order to make labeling with the radionuclide satisfying Equation 1 easier.

In conclusion, Cerenkov imaging showed great potential as a new optical imaging modality. Thanks to the intrinsic optical/PET dual-imaging properties of $^{124}I$, the extra step of conjugating a fluorescent dye that is needed for optical imaging could be omitted, resulting in a simpler but higher yielding preparation of a hybrid nanoprobe for triple-modality imaging. The lymph node metastasis was screened with high sensitivity within a few minutes using an easily accessible optical imager, but the final diagnosis was based on accurate 3D visualization of accumulated nanoprobes with detailed anatomical information provided by PET and MR imaging. This facile method for the preparation of multimodality imaging probes based on Cerenkov imaging has a promising potential for application in many other biomedical and clinical research fields such as cell trafficking, tumor imaging and theragnosis.

According to another embodiment of the present disclosure, it is possible to monitor pure $\beta^+$- or $\beta^-$-emitting radionuclides by non-invasive optical imaging using the contrast agent of the present disclosure. Thus far, due to the lack of $\gamma$-ray emission during decay, pure $\beta^-$ emitters, such as $^{32}P$ and $^{90}Y$, could be radioassayed using only invasive methods, such as liquid scintillation and Cerenkov counting, in which the measurements were performed with extracts. As an example, the phosphate uptake pattern in a plant (arabidopsis) was imaged in real time in luminescence mode, using $^{32}P$-labeled phosphoric acid [$^{32}P$]phosphoric acid (Fig. 14, A-C). Fig. 14, B shows a luminescence image at 10 minutes after immersion of the root. In 10 minutes, a sufficient amount of $^{32}P$ was carried to the leaves. The luminescence intensity at the leaves increased linearly up to 1 hour (Fig. 14, C). Uptake and metabolism of not only phosphorus (phosphates) but also many other inorganic nutrients can be monitored by noninvasive real-time optical imaging, using $^{42}K$, $^{22,24}Na$, $^{86}Rb$, $^{36}Cl$, $^{59}Fe$ or $^{64,67}Cu$. The optical imaging may be performed in the dark. Also, in addition to plants, the real-time optical imaging using the contrast agent of the present disclosure is applicable to animals, including human, zebrafish, etc.

In another embodiment, the present disclosure provides a contrast agent for optical imaging comprising: a radionuclide which emits a charged particle having energy with a threshold T satisfying Equation 1 during radioactive decay; and a fluorophore, wherein the energy emitted from the radionuclide is accumulated in the fluorophore and light is emitted from the fluorophore:

[Equation 1]

$$T\ (\mathrm{keV}) = 511\ [1 / (1 - 1/n^2)^{1/2} - 1]$$

where n is the refractive index of a medium.

Descriptions about Equation 1 and the radionuclide satisfying the equation will be omitted, since they are the same as described above. The existing imaging agent requires the attachment of, for example, a fluorophore to a

substrate for optical imaging even if it already includes a radionuclide. In that case, the procedure of irradiating light in the UV or visible region was needed to excite the fluorophore prior to optical imaging. In contrast, the radionuclide according to the present disclosure satisfying Equation 1 can autoluminesce without external energy input since the fluorophore can absorb the lilght emitted from the radionuclide and reaches the excited state. Specifically, as can be seen from Fig. 17, a combination of $^{124}I$ and QD800 (fluorophore) resulted in a much superior luminescence intensity under a light-blocked environment, as compared to individual $^{124}I$ or QD800. The luminescence intensity was higher than the sum of the luminescence intensities of $^{124}I$ and QD800.

Examples of the fluorophore that can be used in the present disclosure may include fluorescein, rhodamine, Lucifer yellow, B-phycoerythrin, 9-acridine isothiocyanate, Lucifer yellow VS, 4-acetamido-4'-isothiocyanatostilbene 2,2'-disulfonic acid, 7-diethylamino-3-(4'-isothiocyanatophenyl)-4-methylcoumarin, succinimidylpyrene butyrate, 4-acetamido-4'-isothiocyanatostilbene 2,2'-disulfonic acid derivatives, LC™-Red 640, LC™-Red 705, Cy5, Cy5.5, lissamine, isothiocyanate, erythrosin isothiocyanate, diethylenetriamine pentaacetate, 1-dimethylaminonaphthyl 5-sulfonate, 1-anilino-8-naphthalene sulfonate, 2-p-touidinyl-6-naphthalene sulfonate, 3-phenyl-7-isocyanatocoumarin, 9-isothiocyanatoacridine, acridine orange, 9-(isothio-(2-benzoxazolyl)phenyl)maleimide, benzoxadiazole, stilbene, pyrene, fluorophore-containing silica, a group II/IV semiconductor quantum dot, a group III/V semiconductor quantum dot, a group IV semiconductor quantum dot, or a bi- or multi-hybrid structure. Specifically, the fluorophore may be one or more selected from a group consisting of a quantum dot nanoparticle, Cy3.5, Cy5, Cy5.5, Cy7, indocyanine green (ICG), Cypate, ITCC, NIR820, NIR2, IRDye78, IRDye80, IRDye82, cresyl violet, Nile blue, Oxazine 750, Rhodamine 800, lanthanides and Texas red. Most specifically, the fluorophore may be a quantum dot nanoparticle of a group II-VI or group III-V compound. In this case, the quantum dot nanoparticle may be one or more selected from a group consisting of CdSe, CdSe/ZnS, CdTe/CdS, CdTe/CdTe, ZnSe/ZnS, ZnTe/ZnSe, PbSe, PbS, InAs, InP, InGaP, InGaP/ZnS and HgTe. The molar ratio of the radionuclide to the fluorophore may be from 1 : 1000 to 1000 : 1, but the present disclosure is not limited thereto.

Further, the present disclosure provides a contrast agent for optical imaging comprising: the autoluminescent radionuclide of the present disclosure; and at least one of a bioactive substance or a chemical active substance. Specifically, the bioactive substance or the chemical active substance may be selective for or specific to a targeted organ or tissue, and may further comprise the fluorophore described above.

The bioactive substance or chemical active substance may be a hormone, an amino acid, a peptide, a peptidomimetic, a protein, a nucleoside, a nucleotide, a nucleic acid, an enzyme, a carbohydrate, a glycomimetic, a lipid, albumin, a monoclonal or polyclonal antibody, a receptor, an encapsulation compound such as cyclodextrin, or a receptor binding molecule. Specific examples of the bioactive substance or chemical active substance include: steroid hormones for the treatment of breast and prostate lesions; somatostatin, bombesin, cholecystokinin (CCK) and neurotensin receptor binding molecules for the treatment of neuroendocrine tumors; CCK receptor binding molecules for the treatment of lung cancer; ST receptor and carcinoembryonic antigen (CEA) binding molecules for the treatment of colorectal cancer; dihydroxyindolecarboxylic acid and other melanin producing biosynthetic intermediates for the treatment of melanoma; integrin receptor and atherosclerotic plaque binding molecules for the treatment of vascular diseases; and amyloid plaque binding molecules for the treatment of brain lesions. Other examples of the bioactive substance or chemical active substance include synthetic polymers, such as polyaminoacids, polyols, polyamines, polyacids, oligonucleotides, aborols, dendrimers, and aptamers.

In an embodiment of the present disclosure, the bioactive substance or chemical active substance may be selected from nanoparticles, antibodies (e.g., NeutroSpect®, Zevalin® and Herceptin®)), proteins (e.g., TCII, HSA, annexin and Hb), peptides (e.g., octreotide, bombesin, neurotensin and angiotensin), nitrogen-containing simple or complex carbohydrates (e.g., glucosamine and glucose), nitrogen-containing vitamins (e.g., vitamin A, B1, B2, B12, C, D2, D3, E, H and K), nitrogen-containing hormones (e.g., estradiol, progesterone and testosterone), nitrogen-containing active pharmaceuticals (e.g., celecoxib or other nitrogen-containing NSAIDs, AMD3100, CXCR4 and CCR5 antagonists) or nitrogen-containing steroids.

As described above, the contrast agent according to the present disclosure may comprise various bioactive substances or chemical active substances. To increase, for example, specificity for a particular target tissue, organ receptor or other biologically expressed composition, multiple bioactive substances or chemical active substances may be utilized. In such instances, the bioactive substances or chemical active substances may be the same or different. For example, a single contrast agent may comprise multiple antibodies or antibody fragments, which are directed against desired antigens or haptens. Typically, the antibodies used in the contrast agent may be monoclonal antibodies or antibody fragments that are directed against desired antigens or haptens. Thus, for example, the contrast agent may comprise two or more monoclonal antibodies having specificity for a desired epitope and thereby increasing concentration of the contrast agent at the desired site. Similarly and independently, the contrast agent may comprise two or more different bioactive substances or chemical active substances each of which is targeted to a different site of the same target tissue or organ. By utilizing multiple bioactive substances or chemical active substances in this manner, the contrast agent may be advantageously concentrated at several areas of the target tissue or organ, thereby potentially increasing the effectiveness of therapeutic treatment. Further, the contrast agent may have a certain ratio of the bioactive

substance or chemical active substance designed to concentrate the contrast agent at a target tissue or organ such that the desired therapeutic and/or diagnostic results may be optimally achieves.

The contrast agent for optical imaging of the present disclosure is able to perform radiation therapy and diagnosis at the same time. By injecting a contrast agent for optical imaging comprising a radionuclide emitting $\beta^-$ ray or $\alpha$ particles into a living organism, optical images may be obtained through luminescence resulting from the electrons or $\alpha$ particles emitted with sufficient energy during decay while treating a tumor or a disease, which may be utilized to monitor, for example, the therapeutic effect.

In another embodiment, the present disclosure provides a pharmaceutical composition comprising the contrast agent of the present disclosure and a pharmaceutically acceptable carrier. Specifically, the pharmaceutical composition according to the present disclosure may comprise a contrast agent, which forms a complex with a metal, dispsersed in a pharmaceutically acceptable carrier. The pharmaceutically acceptable carrier (also known as excipient, vehicle, adjuvant or diluent in the related art) is usually a pharmaceutically inactive substance, providing adequate hardness or shape to the composition without negatively affecting the therapeutic and/or diagnostic effect of the contrast agent. In general, the carrier is regarded as "pharmaceutically or pharmacologically acceptable" unless it leads to adverse reactions, allergic reactions or other undesired reactions when administered to a mammal, especially human.

The selection of the pharmaceutically acceptable carrier is likely to be affected at least in part by the targeted administration route. In general, the pharmaceutical composition according to the present disclosure may be formulated to be applicable to any administration route allowing access to the target tissue. For example, adequate administration routes may include oral, parenteral (e.g., intravenous, intraarterial, subcutaneous, rectal, intramuscular, intraorbital, intracapsular, intramedullary, intraperitoneal or intrasternal), topical (e.g., nasal, transdermal or intraocular), intravesical, intrathecal, intraabdominal, intrapulmonary, intralymphatic, intracavitary, intravaginal, transurethral, intradermal, intratympanic, intrabreast, buccal, in situ, endotracheal, intraleisonal, endoscopic, transmucosal or sublingual routes.

Pharmaceutically acceptable solvents that can be used in the present disclosure are known to those skilled in the art. For example, The Chemotherapy Source Book (Williams & Wilkens Publishing) may be referred to.

The administration dosage of the contrast agent and/or the pharmaceutical composition according to the present disclosure may be determined easily by those skilled in diagnosis or treatment of diseases. The administration dosage of the contrast agent will be different depending on the age, sex, general health and body weight of the subject, the kind of treatment accompanied, the number of treatments, if any, and the desired effect. Given a specific mode of administration, the actual amount of the delivered contrast agent as well as the administration scheme needed to attain the desired effect will be different, at least in part, depending on the bioavailability of the contrast agent in a living organism, hindrance to treatment or diagnosis, administration dosage for the desired treatment or diagnosis, and other factors apparent to those skilled in the art. The administration dosage for an animal, especially human, should be sufficient enough to exhibit a desired therapeutic or diagnostic effect over an appropriate period.

The present disclosure provides a contrast agent having an adequate radioactivity. In general, it is desired that a radioactive complex is formed in a solution having a radioactivity concentration of about 0.01-1000 mCi/mL. In general, a unit administration dosage may have a radioactivity of about 0.01-1000 mCi, specifically about 1-300 mCi. The volume of a solution injected as a unit administration dosage is about 0.01-20 mL.

A linker may be used to attach the radionuclide with the bioactive substance or chemical active substance. The linker may be a compound represented by Chemical Formula 1, but is not limited thereto:

[Chemical Formula 1]

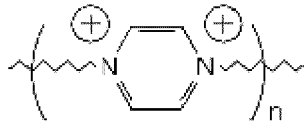 ,

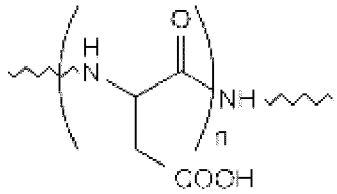 ,

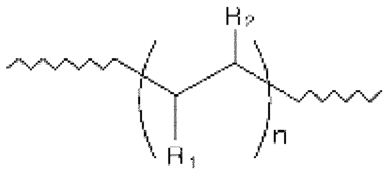 ,

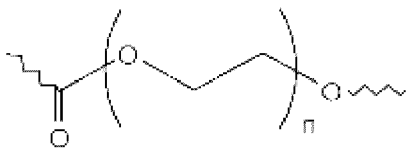 ,

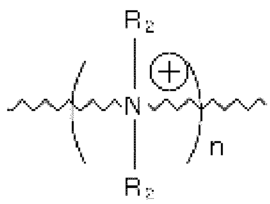 ,

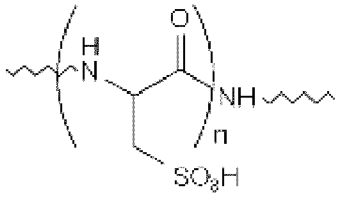 ,

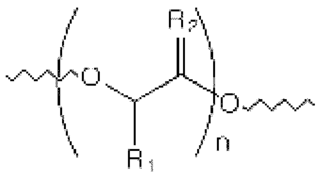 ,

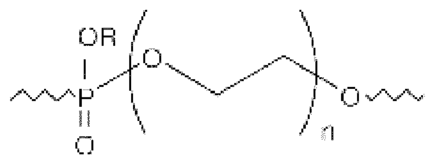 ,

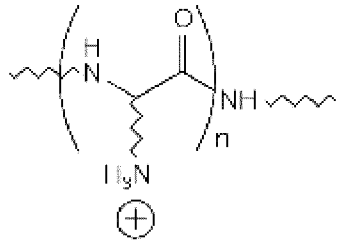 ,

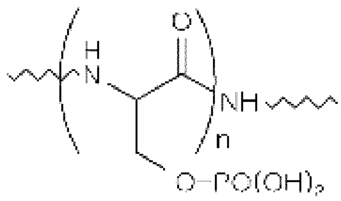 ,

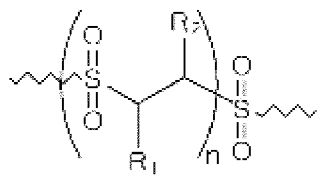 ,

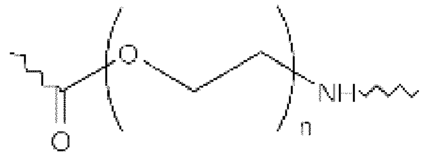 ,

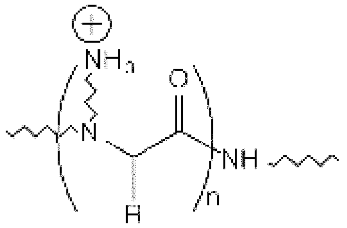, 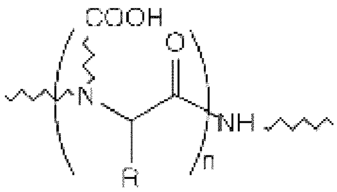, 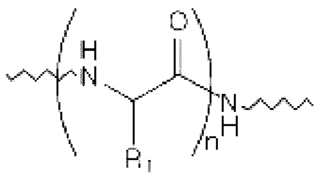,

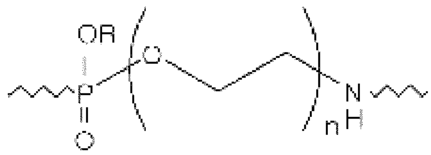, 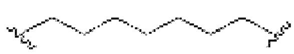, 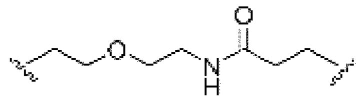,

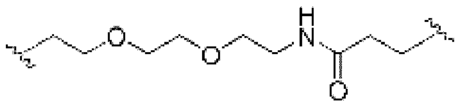, 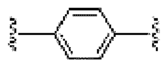, 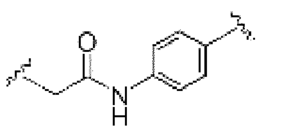,

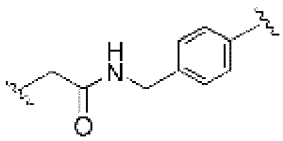

where R, $R_1$ and $R_2$ are independently $C_{1\sim10}$ alkyl, $C_{1\sim10}$ alkenyl, $C_{1\sim10}$ alkynyl, $C_{1\sim10}$ aryl, $C_{1\sim10}$ arylalkyl or $C_{1\sim10}$ heteroaryl, and n is independently an integer from 1 to 20.

As described earlier, the contrast agent for optical imaging of the present disclosure may be used in existing apparatuses for acquiring optical images. More desirably, the present disclosure provides an apparatus for acquiring optical images by detecting light emitted from the contrast agent for optical imaging according to the present disclosure in order to more clearly visualize Cerenkov light, comprising: a chamber accommodating a subject containing the contrast agent for optical imaging; a conversion means provided in the chamber and converting the energy of a charged particle emitted from the contrast agent for optical imaging into light; and a light detection means detecting the light converted by the conversion means. To describe specifically the features of the apparatus referring to Fig. 1, an apparatus 100 for acquiring optical images of the present disclosure may comprise: a chamber 160; a supporting means 140 provided in the chamber 160 and supporting a subject; a conversion means 130 converting the energy of a charged particle emitted from the contrast agent for optical imaging included in the subject into light; and a light detection means 110 detecting the light converted by the conversion means 130 and converting it into an optical image.

Specifically, the subject may contain the contrast agent for optical imaging of the present disclosure therein. The subject may be one commonly used to acquire an optical image without particular limitation. Specifically, it may be a living organism, a tissue, a TLC plate, a gel, a sample, and so forth.

The conversion means 130 serves to convert the energy of a charged particle emitted from the contrast agent for optical imaging included in the subject into light. Specifically, it is the medium of Equation 1. The conversion means 130 may be provided at any position where the light emitted from the subject can reach, without particular limitation. Specifically, it may be provided in contact with or very close to the subject. The conversion means 130 may also be provided on the supporting means 140. As described earlier referring to Equation 1, since the intensity of Cerenkov radiation is proportional to the refractive index of the medium, the higher the refractive index of the conversion means 130, the easier it is to convert the energy of the charged particle emitted from the contrast agent for optical imaging included in the subject into Cerenkov light. Specifically, if the subject is a simple sample, the conversion means 130 may have a refractive index higher than that of air (1.0003). If the subject is a living organism (e.g., mouse or human), the conversion means 130 may have a refractive index higher than that of water (1.33), more specifically, 1.50 or higher. Although glass having a refractive index of 1.52 was used as the conversion means in the examples related to Figs. 13 and 14, the present disclosure is not limited thereto. Any medium having a high refractive index and made of a material through which light can pass may be used without limitation.

Specifically, the conversion means 130 may be in the form of a sheet. The thickness may be 0.1-20 mm, but is not limited thereto. The number of the conversion means 130 may be one or more. The conversion means 130 may

be configured to be separable as occasion demands and may be configured to be on/off controllable. In addition, it may be configured to have a variable refractive index using, for example, a filter. Also, the apparatus may be configured to capture the light emitted from the subject directly without using the conversion means.

The light detection means 110 serves to detect the light converted by the conversion means 130 and convert it into an optical image, and may comprise a photographing or imaging device commonly used in an apparatus for acquiring optical images. Specifically, the light detection means 110 may comprise a lens and/or a filter 120 through which the light converted by the conversion means 130 passes and a charge-coupled device (CCD) camera 111 or a photomultiplier tube (PMT) for converting the light into an optical imaging. However, without being limited thereto, the light detection means commonly used in an apparatus for acquiring optical images may be used without limitation.

The chamber 160 may be one through which light cannot penetrate. The chamber may be made of a radiation-shielding lead or tungsten material, or may be equipped with a shielding means. However, the present disclosure is not lilmited thereto. The apparatus for acquiring optical images of the present disclosure may be used as an apparatus for luminescence imaging, fluorescence imaging, bioluminescence imaging, radioluminescence imaging or a combination thereof. However, without being limited thereto, it may be utilized without limitation as long as the principle of the present disclosure is applicable.

Further, in case the subject is an animal, an anesthetic means 150 for anesthizing may be included in the apparatus for acquiring optical images of the present disclosure.

Operation of the apparatus for acquiring optical images according to the afore-described embodiemnt of the present disclosure will be described. First, a subject is positioned on the supporting means 140. The subject contains (through injection) the contrast agent for optical imaging of the present disclosure. Thus, a charged particle (e.g., electron or positron) having energy (kinetic energy) is emitted from the contrast agent for optical imaging included in the subject. If the emitted charged particle satisfies Equation 1, it has a velocity greater than that of speed of light. Then, the charged particle is decelerated to the speed of light while passing through the conversion means 130, and the corresponding energy difference is emitted as light. Specifically, the conversion means 130 may be made of a material having a high refractive index in order to reduce the minimum energy T required to emit Cerenkov light. The light converted by the conversion means 130 is detected by the light detection means 110 to construct an optical image.

An apparatus for acquiring optical images for medical use according to another embodiment of the present disclosure will be described referring to Fig. 2. Specifically, an apparatus 200 for acquiring optical images for medical use according to another embodiment of the present disclosure by detecting light emitted from the contrast agent for optical imaging according to the present disclosure comprises: a chamber 210; a supporting means 240 provided in the chamber 210 and supporting a subject containing the contrast agent for optical imaging; a conversion means 230 provided in the chamber 210 and converting the energy of a charged particle emitted from the contrast agent for optical imaging into light; and a light detection means 220 provided in the chamber 210 and detecting the light converted by the conversion means 230. The apparatus for acquiring optical images for medical use according to this embodiment is the same as the apparatus for acquiring optical images according to the afore-described embodiment for general use in the contrast agent used, but is different in that the subject is human. Hereunder, only the difference from the afore-described embodiment will be described.

Specifically, the apparatus 200 for acquiring optical images for medical use may comprise the supporting means 240, the conversion means 230 and the light detection means 220 inside the chamber 210. At least one of the supporting means 240 and the light detection means 220 may be configured to be freely movable relative to each other by providing a position control means. The position control means may be any one that can move the supporting means 240 or the light detection means 220 upward, downward, leftward and rightward. Accordingly, the subject positioned on the supporting means 240 may be examined wholly by moving the supporting means 240 or the light detection means 220. Also, the conversion means 230 may be moved together with the light detection means 220 or the supporting means 240.

The light detection means 220 may be brought in close proximity to the desired site (e.g., thyroid) of the subject using the position control means so as to acquire optical images. The chamber 210 may be one through which light cannot pass. The chamber itself and/or the light detection means 220 may be shielded from radiation. The supporting means 240 may have any shape as long as it is capable of supporting, for example, human, including bed or chiar shapes.

According to another embodiment of the present disclosure, the apparatus for acquiring optical images of the present disclosure may be an endoscope. Specifically, referring to Fig. 3, an endoscope 300 for acquiring optical images by detecting light emitted from the contrast agent for optical imaging according to another embodiment of the present disclosure comprises: a light source 320 for illuminating the inside of a subject; a conversion means 330 converting the energy of a charged particle emitted from the contrast agent for optical imaging, which is contained in the subject, into light; and a light detection means 360 detecting the light converted by the conversion means 330. The endoscope according to this embodiment is the same as the apparatus for acquiring optical images according to the afore-described embodiment and the contrast agent used therein in basic configuration and operation principle. Hereunder, only the difference from the afore-described embodiments will be described.

The endoscope 300 may be inserted into a living organism. The living organism has the contrast agent for

optical imaging of the present disclosure injected therein. Since the living organism is light-shielded in itself, an additional means to block light penetration into the endoscope 300 is not necessary. Differently from the apparatus for acquiring optical images according to the afore-described embodiment, the endoscope 300 comprises the light source 320 for illuminating the inside of the living organism. The light source 320 may be a lamp. It may be provided at a leading end portion of the endoscope 300, but its location is not limited thereto. And, the conversion means 330 for converting the energy of the charged particle into light in the living organism may be provided either in front of the light source 320 or between the light source 320 and the light detection means 360. Most specifically, it may be provided at the leading end portion of the endoscope, i.e. in front of the light source 320, so as to convert the energy of the charged particle emitted from the contrast agent for optical imaging injected into the living organism at a short distance. A body 310 of the endoscope 300 has the same configuration as the commonly used endoscope. It may include a means (e.g., optical fiber) to transmit the light converted by the conversion means 330 to the light detection means 360.

The principle of converting the energy of the charged particle emitted from the contrast agent for optical imaging, which is contained in the subject, into light using the conversion means 330 and then converting the converted light into an optical image using the light detection means 360 is the same as that described in the above embodiment.

Now, a radionuclide detection apparatus 400 according to an embodiment of the present invention will be described referring to Fig. 4. In the embodiment, the present disclosure provides a radionuclide detection apparatus 400 for detecting a radionuclide labeled at a subject exisiting in a fluid flowing in a tube 450, which comprises: a chamber 440 through which the tube 450 penetrates; a conversion means 430 provided in the chamber 440 and converting the energy of a charged particle emitted from the radionuclide into light; and a light detection means 410 detecting the light converted by the conversion means 430.

The radionuclide detection apparatus according to this embodiment is the same as the apparatus for acquiring optical images according to the above-described embodiments in basic configuration and operation principle. Hereunder, only the difference from the afore-described embodiments will be described.

The tube 450 may have a diameter of 0.1-10 mm, although not being limited thereto. A fluid passes inside the tube 450. The fluid contains the subject labeled with the contrast agent for optical imaging of the present disclosure. The subject may be any subject that can be subjected to high performance liquid chromatography (HPLC). Specifically it may be antibody, protein, antigen, peptide, nucleic acid, enzyme, cell, carbohydrate, vitamin, hormone, nanoparticle, inorganic support, polymer, single molecule or drug, but is not limited thereto.

The tube 450 penetrates the chamber 440. The tube 450 has a light transmitting portion 460, so that the light emitted from the subject can be transmitted through the chamber 440. The light transmitting portion 460 is made of any transparent material allowing penetration of light, without particular limitation. The light transmitting portion 460 is formed at the position facing the conversion means 430.

The conversion means 430 converts the energy of the charged particle passing through the light transmitting portion 460 into light. The light converted by the conversion means 430 is then converted into an optical image by the light detection means 410.

Now, operation of the radionuclide detection apparatus according to this embodiment will be described. The fluid containing the subject flows in the tube 450. The tube 450 penetrates the chamber 440. The charged particle emitted from the radionuclide labeled at the subject passes through the light transmitting portion 460. Then, the energy of the charged particle is converted into light by the conversion means 430. The converted light is then converted into an optical signal by the light detection means 410. Specifically, in another embodiment, the light transmitting portion 460 may be made of a material with a high refractive index (e.g., glass or transparent plastic), so that it may perform the photoconversion even without the conversion means 430.

Unlike the existing apparatus for acquiring optical images, the apparatus for acquiring optical images and the radionuclide detection apparatus of the present disclosure may comprise the conversion means capable of converting the energy of the charged particle emitted from the radionuclide included in the subject into Cerenkov light and/or may comprise the radiation shielding means for protecting the chamber and/or the light detection means from the radionuclide.

The conversion means may be made of a material having a high refractive index. The apparatus for acquiring optical images and the radionuclide detection apparatus may be utilized widely as an apparatus for acquiring optical images for medical or non-medical use, as a radio-detector, for measurement of surface radioactive contamination, as a radiation detector for HPLC, and so forth.

[Mode for Disclosure]

Comparative clarifying examples and experiments will now be described. The following examples and experiments are for illustrative purposes only and not intended to limit the scope of the present disclosure.

<Example 1> Luminescence imaging using [$^{18}$F]FDG

A BALB/c mouse with a luciferase-transfected 4T1 tumor was injected with 409 mCi of [$^{18}$F]FDG via the tail vein. One hour after the injection, the mouse was scanned for 3 minutes in the prone position using IVIS 200. Fig. 5a shows a luminescence image of the BALB/c mouse taken at 1 hour after injecting [$^{18}$F]FDG. The brain (red arrow) and the brown fat (white arrow) are clearly visualized. Thus, it was confirmed that a luminescence image can be obtained using [$^{18}$F]FDG without an additional fluorophore or luminophore.

<Example 2> Luminescence imaging using $^{124}$I

500 mCi of $^{124}$I was injected intraperitoneally into a normal mouse (C3H/HeN, Orient Bio Inc., Korea). 40 minutes after the injection, a luminescence image was obtained by scanning the mouse for 1 minute in the lying position. Fig. 5b is the image of the mouse (C3H/HeN) obtained at 40 minutes post-injection of [$^{124}$I]NaI. The thyroid (white arrow head) and the bladder (red arrow head) can be seen in addition to the snout (yellow arrow head). Thus, it was confirmed that a luminescence image can be obtained using $^{124}$I.

<Example 3> In vitro luminescence imaging

A 96-well plate filled with each of the successively diluted solutions (300 mL) of [$^{124}$I]NaI, [$^{18}$F]FDG, [$^{68}$Ga]GaCl$_3$, [$^{131}$I]NaI and [$^{99m}$Tc]TcO$_4^-$ was scanned for 1 minute using IVIS 200 (Fig. 6a). The high dependence of luminescence intensity on radioisotope species was clearly observed. Whereas the positron emitter $^{68}$Ga showed the most intense signal, the $\gamma$-ray emitter, $^{99m}$Tc, did not show any detectable light signal at similar activity concentrations. The other two positron emitters, $^{124}$I and $^{18}$F, showed the second and third strongest luminescence signals, respectively, and although the luminescence signal of the $\beta^-$ emitter, $^{131}$I, was not as strong as the other three positron emitters, it also showed a sufficiently detectable light signal.

<Example 4> In vitro luminometric assay

2.0, 1.08, 1.84, 3.06, 1.08, 2.20, 2.42 and 3.0 mCi/mL [$^{32}$P]phosphoric acid, [$^{124}$I]NaI, [$^{18}$F]FDG, [$^{131}$I]NaI, [$^{64}$Cu]CuCl$_2$, [$^{111}$In]InCl$_3$, [$^{99m}$Tc]TcO$_4^-$ and [$^{35}$S]methionine, respectively, were diluted successively one third to prepare 10-11 solutions for each radionuclide. A 96-well plate containing 300 mL of a solution of each radionuclide at different concentrations was scanned for 30 seconds at 470 nm using a bench top luminometer (SpectraMax L, MDS Analytical Technologies, USA). The result is shown in Figs. 6b and 6c. The relative intensity of the radionuclides was calculated from the slope of the line of best fit for each nuclide.

A similar relationship between the luminescence intensity and radioisotope species was observed. In general, all the radionuclides showed increase in luminescence intensity with increasing radiation activity. However, three isotopes, $^{111}$In, $^{99m}$Tc and $^{35}$S, showed much slower increase in light signal compared to the other five nuclides, $^{32}$P, $^{124}$I, $^{18}$F, $^{131}$I and $^{64}$Cu. In particular, $^{35}$S did not show any noticeable increase in luminescence even at the highest activity. Whereas the nuclides emitting high-energy electrons and positrons, $^{68}$Ga, $^{32}$P, $^{124}$I, $^{18}$F, $^{131}$I and $^{64}$Cu, showed strong signals, $^{111}$In, $^{99m}$Tc and $^{35}$S showed weak signals.

Therefore, it was confirmed that the luminescence originates from the radionuclides. Also, it was shown that the luminescence of the radionuclides is highly dependent on their decay mode. All the luminescence signals could be blocked completely by covering the well containing the radionuclide with black paper, which suggests that the detected signal orginates from the light emitted from the radionuclide, not from any interaction between the charged particle or gamma ray and the detector.

<Example 5> Luminescence and microPET imaging using radioiodinated Herceptin

A small quantity of Herceptin (trastuzumab) was radiolabeled with $^{124}$I using lodo-Beads (Pierce Biochemical Co., USA) according to the manufacturer's instructions. To put briefly, the beads were washed with PBS (pH 7.2) and dried on filter paper at room temperature. The washed beads were put in a [$^{124}$I]NaI (37 MBq) solution in 100 $\mu$L of PBS. After shaking for 5 minutes, the [$^{124}$I]NaI solution containing lodo-Beads was mixed with an aqueous solution of Herceptin (30 $\mu$g, 30 $\mu$L of distilled water). Labeling was performed for 15 minutes at room temperature while gently shaking. The reaction was terminated by removing the beads from the reaction tube. The labeling yield was monitored on a silica plate using acetone as a developing solvent, using a radio-TLC imaging scanner (Bioscan Inc., Washington DC, USA). Unlabeled free $^{124}$I ions were removed using a centrifugal filter (Microcon YM 50, Millipore Inc., USA), and the purified $^{124}$I-labeled Herceptin (3.29 MBq) was injected into a nude mouse bearing a NIH3T6.7 tumor on the shoulder and flank. The mouse was scanned for 20 min using a microPET at 2 days after the injection, which was immediately followed by

luminescence imaging (scan time = 1 min).

Immediately after the in vivo imaging, internal organs as well as tumors were dissected out, and luminescence and microPET images were obtained with scan times of 1 minute and 20 minutes, respectively. Relative intensity of each organ in the luminescence and microPET images was calculated by quantification of ROI drawn on each excised organ.

Herceptin was easily radioiodinated with $^{124}I$ (3.29 MBq) using the well-established Iodo-Beads method, by which an oxidized $I^+$ ion reacts with the tyrosine residue of the antibody. The radiolabeling yield was 78% and the radiochemical purity was increased to 92% after purification using the centrifugal filter. The NIH3T6.7 tumours implanted in the nude mouse were clearly visualized by $^{124}I$-labeled Herceptin antibody in luminescence imaging (Fig. 7, A). Two tumor lesions were clearly visualized in 1 minute scanning time with minimal background by adjusting the threshold of color scale, which indicates that the radiolabeled Herceptin has high binding affinity for NIH3T6.7 tumors. Because $^{124}I$ is a positron-emitter, the same $^{124}I$-labeled Herceptin could also be imaged using a microPET scanner (Fig. 7, B). Even though the two tumor sites were clearly visualized in optical imaging, only the larger sized tumor (6 $\times$ 5 mm) on the shoulder was barely recognized in the microPET image. However, the internal organs with high uptake of the labelled Herceptin were clearly recognized in coronal image of microPET.

Ex vivo optical and microPET images of the dissected organs and tumors showed different radiation uptake patterns (Fig. 7, C-E). All the organs were visualized with a wide range of contrast in luminescence imaging, while some organs such as muscle with lesser amounts of radiation activity were not recognized clearly in the microPET image. However, the microPET image revealed the actual amount of radiation activity in the organs, thanks to the accurate quantification ability of nuclear imaging. Cerenkov light emitted from the liver and kidneys was very small in quantity considering the high uptake of radiation in these organs, which was confirmed from the microPET imaging, presumably because of the low tissue penetration ability of Cerenkov light in the high blood-rich organs. When each relative luminescence intensity of the dissected organs was compared with that of the microPET image and the relative intensities of the thymus in both images were set to equal, the relative intensities of three blood-rich organs (liver, kidneys and spleen) in PET imaging were 1.5-1.8 times higher than those in optical imaging, while other organs showed slightly higher relative intensity in PET compared to in luminescence imaging except the tumors.

<Example 6> Optical, nuclear and MR imaging using radiolabeled nanoparticles

(1) Synthesis of tyramine-conjugated poly(TMSMA-r-PEGMA-r-NAS)

Poly(TMSMA-r-PEGMA-r-NAS) was synthesized according to the previously reported method. Specifically, as illustrated in Fig. 8a, TMSMA (3.73 mmol, 0.9 g, 1 eq.), PEGMA (3.73 mmol, 1.77 g, 1 eq.) and NAS (3.2 mmol, 0.54 g, 0.86 eq.) were dissolved in 8 mL of THF (anhydrous, 99.9%, inhibitor-free). Then, nitrogen was blown to the mixture solution for 15 minutes in order to remove gas. After adding 2,2'-azobisisobutyronitrile (0.03 mmol, 5 mg, 0.01 eq.) as a radical initiator, polymerization was performed for 24 hours at 70 °C [$^1H$ NMR (300.40 MHz, CDCl3): $\delta$ = 4.14 (br, 2H, $CO_2$-$CH_2$ of PEGMA), 3.98 (br, 2H, $CO_2$-$CH_2$ of TMSMA), 3.66 (s, 30H), 3.63-3.55 (s, 9H; m, 2H), 3.37 (s, 3H), 2.80 (br, 4H, CO-$CH_2$ of NAS), 2.0-1.71 (br, 6H), 1.04 (br, 2H), 0.87 (br, 4H), 0.66 (br, 2H)]. Polydispersity was measured at 2.1 ($M_n$ = 8,861 and $M_w$ = 19,132) by gel permeation chromatography using a Waters 1515 isocratic pump and a Waters 2414 refractive index detector. THF was used as the eultion solvent and the flow rate was 0.4 mL/min. Tyramine (32 $\mu$mol, 4.4 mg in 400 $\mu$L of DMF) was added to the resulting polymer (28 $\mu$mol, 250 mg in 1 mL of THF). 6 hours later, tyramine-conjugated poly(TMSMA-r-PEGMA-r-NAS) was separated using a dialysis membrane (MWCO 100K, Spectrum Laboratories Inc., Rancho Dominguez, CA). The content of tyramine in the poly(TMSMA-r-PEGMA-r-NAS) was measured by UV analysis at 270 nm. The tyramine content in the polymer was calculated as 0.77 wt % (Fig. 8, b)). The hydrodynamic particle size of the tyramine-conjugated TCL-SPION was measured to be 39$\pm$8 nm (Fig. 8, c)).

(2) Synthesis of tyramine-conjugated TCL-SPION

$FeCl_3 \cdot 6H_2O$ (0.5 g, 1.85 mmol) and $FeCl_2 \cdot 4H_2O$ were dissolved in deionized water (30 mL) from which gas had been removed by blowing nitrogen for 30 minutes. Then, 7.5 mL of $NH_4OH$ (-28% water) was added to the solution under nitrogen atmosphere while strongly stirring. 30 minutes later, an external magnetic field was applied. The resulting precipitate was washed with deionized water to collect the black particles. After discarding the supernatant, 250 mg of the tyramine-conjugated poly(TMSMA-r- PEGMA-r-NAS) in 30 mL of deionized water was added, and the mixture solution was stirred for 2 hours. After purification to remove the remaining unreacted polymer, ultrasonic pulverization was performed for 30 minutes in 20 mL of deionized water using a VCX-500 ultrasonic processor (Sonics & Materials, Inc., Newtown, CT). Then, after applying an external magnetic field overnight, the precipitated particles were removed by centrifugation for 10 minutes at 10,000 rpm. The supernatant was diluted with deionized water and then heated for 1.5 hours at 80 °C in order to form cross-linkages beween polymer rings on the particle surface. The resulting thermally

cross-linked tyramine-conjugated superparamagnetic iron oxide nanoparticle (TCL-SPION) was concentrated for animal tests by filtration (Amicon Co., USA). The magnetite core was analyzed by tunneling electron microscopy (TEM) using a TECNAI F20 electron microscope (Philips Lectronic Instruments Corp., Mahwah, NJ) operating at 200 kV. The hydrodynamic particle size of the tyramine-conjugated TCL-SPION was measured as 39.8±8.3 nm using a Photal ELS-8000 electrophoretic light scattering apparatus (Otsuka Electronics Co., Japan). The TEM analysis revealed that the magnetite core size was between 4 and 11 nm. The nanoparticle was dispersed well in aqueous solution and was stable without any aggregation. TCL-SPION and Feridex (control) solutions were prepared at 0.05, 0.1, 0.2 and 0.4 mg Fe/mL concentration. Their T2 relaxivity coefficients ($r_2$) were measured with different echo time in a fast spin echo sequence (repetition time (TR) = 4000 ms, echo time (TE) = 10, 30, 60, 90, 120, 150, 180, 210, 240, 270, 300, 350, 400, 450, 500, 550, 600, 650, 700, 750, 800, 850, 900, 950, 1400, 1500, 1800, and 1900 ms, field of view (FOV) = 9, matrix = 320 × 160, slice thickness = 5 mm).

Fig. 9 shows a spin-spin relaxation rate (R2) of the tyramine-conjugated TCL-SPION versus iron concentration. It shows that the r2 value of TCL-SPION (283.7 $mM^{-1}s^{-1}$) is higher than that of Feridex (234.2 $mM^{-1}s^{-1}$), indicating excellent imaging effect as compared to the commercially available MR contrast agent.

(3) Radiolabeling of tyramine-conjugated TCL-SPION with $^{124}I$

Radiolabeling of the tyramine-conjugated TCL-SPIONs with $^{124}I$ was easily accomplished using commercially available lodo-Beads (Pierce Biochemical Co., USA) according to the manufacturer's instructions. To put briefly, the beads were washed with PBS and dried on filter paper at room temperature. The washed beads were put in a [$^{124}I$]NaI (1.85 mCi) solution in 100 μL of PBS. After shaking for 5 minutes, the [$^{124}I$]NaI solution containing lodo-Beads was mixed with an aqueous solution of the nanoparticle (152 μg, 20 μL of distilled water). Labeling was performed for 15 minutes at room temperature while gently shaking. The reaction was terminated by removing the beads from the reaction tube. The labeling yield was monitored on a silica plate using acetone as a developing solvent, using a radio-TLC imaging scanner (Bioscan Inc., Washington DC, USA). The radiolabeling yield was 79%. Unlabeled free $^{124}I$ ions were removed using a centrifugal filter (Microcon YM 50, Millipore Inc., USA). The radiochemical purity of was 92% after the purification.

(4) Comparision of optical, microPET and MR imaging

a. To evaluate the sensitivity of Cerenkov luminescence imaging, a series of solutions (300 μL) containing $^{124}I$ and TCL-SPION were imaged by optical, microPET and MR imaging (Fig. 10, a)-c)). A 1 mL solution containing 762 μCi of [$^{124}I$]NaI and 205 μg of the tyramine-conjugated TCL-SPION was diluted successively one third to prepare 10 solutions for luminescence, microPET and MR imaging. A 96-well plate containing 300 μL of each diluted solution was scanned for 1 minute using IVIS 200. The same solutions were microPET scanned (Inveon Imaging System, Siemens, Germany) for 20 minutes. MR imaging was performed at 1.5 T in a T2-weighted fast spin echo sequence (repetition time (TR) = 3000 ms, echo time (TE) = 87 ms, matrix = 256 × 256, field of view (FOV) = 10 × 10 cm, slice thickness = 2 mm).

Referring to Fig. 10, a)-c), one of the most noticeable features of Cerenkov imaging in comparison with microPET and MR imaging is the wide range of contrast in the luminescence images. A range of activity concentrations varying from 762 to 3.14 μCi/mL $^{124}I$ was clearly visualized and, more importantly, the concentrations were differentiated from each other. The sensitivity and contrast of Cerenkov imaging were notable, given the short imaging time of optical imaging (1 min) versus that of microPET and MR imaging (20 min for both). In the MR images, the solutions containing up to 0.84 μg/mL TCL-SPION showed noticeable contrast compared to blank water, but consideration of the 3.0 ng mass of 762 μCi of $^{124}I$ clearly indicated the much lower sensitivity of MRI compared to that of Cerenkov imaging and PET imaging. Furthermore, the three most concentrated solutions could not be differentiated from each other in the MRI.

b. The spatial resolution of Cerenkov imaging was studied using a Derenzo phantom having holes of different sizes filled with radioactive solutions. The core part of the Derenzo phantom was filled with a [$^{124}I$]NaI solution (254 μCi in 8 mL distilled water). The luminescence and microPET images were obtained for 1 minute and 20 minutes, respectively. The PET images were reconstructed using the ordered subset expectation maximization 3D-MAP algorithm. The Derenzo phantom containing 32 μCi of $^{124}I$ was imaged by luminescence and microPET imaging (Figure 10, d) and e)). Cerenkov imaging could clearly identify each source up to a resolution of 1.6 mm and some sources could be separated from other nearby sources with a resolution of 1.2 mm, while a maximum resolution of 2.4 mm was achieved in microPET imaging. This demonstrated the higher spatial resolution of optical imaging based on Cerenkov radiation compared to microPET imaging, at least for in vitro imaging.

c. Animal experiments were conducted to investigate tissue penetration of Cerenkov imaging. A series of different

concentrations of [$^{124}$I]NaI in 10 $\mu$L volumes was injected into the back muscle (longissimus muscle group of the thoracic and lumbar vertebrae region) of Sprague-Dawley rats (ca. 420 g, Orient Bio Inc., Korea) at depths of 4 mm and 7 mm with increasing concentrations. The luminescence images were obtained using IVIS 200 after each injection for 1 minunte. The rats were sacrificed by cervical dislocation just before radioactivity injection to prevent the fast diffusion of the injected radiation into the surrounding tissue, and the hair around the injection area was removed. All the luminescence images were obtained using IVIS 200 (Caliper Life Sciences, Inc., USA) in bioluminescence mode, with no excitation light source or emission filter.

(5) Triple-modality imaging of radiolabeled nanoparticles

Triple-modality images were obtained for a tumor metastasis model using the contrast agent for trimodality imaging prepared in Example 6, (3). Specifically, mouse breast cancer cell line 4T1 was cultured in RPMI 1640 containing 10% FBS and 1% antibiotics (penicillin and streptomycin) and maintained at 37 °C in a 5% $CO_2$ incubator. The 4T1 tumor cells ($1\times10^7$) were injected into the left shoulder of normal BALB/c mice (female, Orient Bio Inc., Korea). After 9 days, the tumor size grew to about 144 mm$^3$. $^{124}$I-labeled nanoparticles (38 $\mu$g, 20 $\mu$Ci) were subcutaneously injected into both front paws of the 4T1 tumor-bearing BALB/c mouse, and microPET imaging was performed for 30 minutes. MR imaging was performed successively at 1.5 T (GE Signa Excite 1.5T, USA) in a T2-weighted fast spin echo sequence. Luminescence imaging was performed for 1 minute. The dissected sentinel lymph node was microPET imaged for 20 minutes and then scanned for 3 minutes using IVIS 200.

As seen from Fig. 11, all the three imaging modalities consistently showed a much lower uptake of the injected nanoparticles in the left brachial sentinel lymph node located close to the tumor site, presumably due to the metastasis of the 4T1 tumor cells into the closer lymph node and the destruction of the lymph node function. In contrast, Cerenkov imaging was superior to the other imaging modalities in terms of its easy identification of the lymph nodes with minimal background and anatomical information supported by photographic images (Fig. 11, a)). The autofluorescence background is one of the shortcomings of the fluorescence imaging modality. However, Cerenkov imaging, being a type of luminescence imaging, does not suffer this annoying optical interference because it does not need any external light source, as in the case of bioluminescence imaging. In addition to the injection sites and the lymph nodes, the signal was also seen in the bladder region, presumably due to the urinal excretion of the co-injected unpurified free radioiodine. Because the microPET image was reconstructed based on the signal from $^{124}$I, the image was well matched with the Cerenkov imaging results. Even though the precise location of one strong signal around the right brachial lymph node was not clear in the PET images because of its poor anatomical information, the microPET imaging offered the advantage of accurate visualization of the radioactivity distribution in the internal organs, thanks to its excellent tissue penetration nature (Fig. 11, b)). The strong dark spot could be unambiguously assigned to the brachial lymph node on the basis of the detailed tomographic anatomical information provided by MRI (Fig. 11, c)). The microPET-MR fusion image guided by fiducial markers revealed the perfect match of the strong blue spot in the microPET image with the intense dark spot in the MR image. The ex vivo optical and microPET images of the dissected lymph node were well matched to the in vivo imaging results (Fig. 11, d)). The immunostaining studies of the excised lymph nodes confirmed that the sentinel lymph node close to the implanted tumor was actually metastasized by the 4T1 tumor cells and had a lower uptake of the iron-based nanoparticles.

Fig. 12 shows a microPET/MR fusion image. The PET/MR fusion image (c) was well matched with a superposition of the microPET (a) image and the MR image (b). During the PET and MR imaging, an animal-specific positioning frame fabricated using paper clay was used to provide reproducible positioning of the mouse. Four small pipette tips (white arrow heads) containing [$^{124}$I]NaI solution were fixed around the mouse as fiducial markers. The PET/MR fusion image was acquired manually using the AMIDE software, with the four fiducial markers as references. The strong green spots around the brachial lymph node in the PET image perfectly matched with the black spots in the MR image. The lymph nodes and the injection sites were denoted as red dotted circles and white I's, respectively.

(6) Histological examination and immunohistochemistry

After the optical imaging in Example 6, (5), the mice were anesthetized using isoflurane/oxygen mixture gas. The lymph nodes and tumors were excised surgically, fixed using 10% neutral formalin, embedded in paraffin wax, and then stained with hematoxylin and eosin (H&E) and Prussian blue. For immunohistochemistry, intrinsic peroxidase activity was inhibited using 3% hydrogen peroxide. Then, antigens were retrieved with microwaves using 10 mmol/L citrate. Anti-cytokeratin 8 and 18 (CK8/18, Novocastra Laboratories, UK) were used for the immunostaining. The antigen-antibody complex was visualized by an avidin-biotin peroxidase complex solution using the ABC kit (Vector Laboratories, USA). Commercially available 3,3'-diaminobenzidine (Zymed, USA) was as a chromogen used for color development. The cells stained with Mayer's hematoxylin were counted.

Prussian blue staining is a method commonly used to detect $Fe^{3+}$ in tissues. Prussian blue binds with $Fe^{3+}$ in

the tissue to produce bright blue color. Since TCL-SPION is an iron oxide ($Fe^{3+}$)-based nanoparticle, the presence of TCL-SPION in the tissue can be detected as bright blue color through Prussian blue staining. CK8/18 immunohistochemistry has been employed to detect epithelial-derived tumor cells. CK8/18 is expressed in high levels in the cytoplasm of tumor cells. A lot of tumor cells were metastasized to the left brachial lymph node and, hence, uptake of TCL-SPION was low. However, in the right brachial lymph node, only a few tumor cells were observed, and uptake of TCL-SPION was much higher in the right brachial lymph node than the left (Fig. 13).

In conclusion, Cerenkov imaging showed great potential as a new optical imaging modality. Thanks to the intrinsic optical/PET dual-imaging properties of $^{124}I$, the extra step of fluorophore conjugation that is needed for optical imaging could be skipped, resulting in the simpler but higher yielding preparation of a hybrid nanoprobe for triple-modality imaging. The lymph node metastasis was screened with high sensitivity within a few minutes using an easily available optical imager, but the final diagnosis was based on accurate 3D visualization of accumulated nanoprobes with detailed anatomical information provided by PET and MR imaging. This facile method for the preparation of multimodality imaging probes based on Cerenkov imaging has a promising potential for application in many other biomedical and clinical research fields such as cell trafficking, tumor imaging and theragnosis.

<Example 7> Luminescence imaging of plant

Prior to luminescence imaging, mature arabidopsis *(Arabidopsis thaliana,* Columbia-O ecotype) had been kept in the dark for 60 minutes. The root of arabidopsis was immersed in a [$^{32}P$]phosphoric acid (37 MBq/5 mL) solution and in $^{32}P$-free water (control). Immediately thereafter, a dynamic luminescence scan was performed with 2-minute exposure at every 5 minutes for 1 hour. The luminescence signal from the aqueous solution was blocked with black paper-covered aluminum foil. The luminescence intensity of selected leaves was quantified as a function of time.

The absorption pattern of phosphate by the plant was imaged in real time using [$^{32}P$]phosphoric acid in luminescence mode (Fig. 14). Fig. 14, B shows the luminescence image of the plant leaves 10 minutes after immersing the root into the [$^{32}P$]phosphoric acid solution and water. Within 10 minutes, sufficient $^{32}P$ was transported to all the leaves and luminescence light was emitted. In contrast, signals were hardly detected from the plant immersed in water. The luminescence intensity of the leaves increased linearly until 1 hour for the $^{32}P$ solution. In contrast, the intensity decreased slightly for the leaves of the control group (Fig. 14, C).

<Example 8> Luminescence images of sequentially diluted [$^{99m}Tc$]$TcO_4^-$ solutions

Luminescence images of sequentially diluted [$^{99m}Tc$]$TcO_4^-$ solutions (300 $\mu$L/well) were obtained for 5 minutes after blocking the wells containing radionuclides of different concentrations using black opaque paper. Only the 2.9 mCi/mL solution emitted very faint light (Fig. 15).

<Example 9> Comparison of luminescence intensities between [$^{18}F$]FDG and free [$^{18}F$]KF ion

[$^{18}F$]FDG and [$^{18}F$]KF having similar radioactivity in 300 $\mu$L of an aqueous solution were diluted sequentially by one third. The light intensity was measured using an in vitro luminometer. The relationship between the relative light unit (RLU) and radioactivity was plotted, and a straight line was drawn using the least-squares method for both radionuclides. There was no significant difference in luminescence between [$^{18}F$]FDG and [$^{18}F$]KF (Fig. 16).

Thus, it was shown that, although [$^{18}F$]FDG and [$^{18}F$]KF are different in chemical forms with the former being a compound and the latter ion, the two do not exhibit significant difference in luminescence intensity.

<Example 10> Measuerement of luminescence emission spectra using $^{124}I$ and quantum dots

The emission spectra were measured using FluoroLog-3 (Horiba Jobin Yvon Inc. USA). 3 mL of an aqueous solution of [$^{124}I$]NaI (2.75 mCi) in a quartz cuvette was scanned before and after adding 10 $\mu$L of quantum dots (Qdot® ITK™ 800, Invitrogen, USA), which emit maximum fluorescence at 800 nm. The scan time was 1 second per 1 nm wavelength with a total of 40 scans. Quantum dots of the same concentration were scanned in the same manner as the control. The luminescence intensity of [$^{124}I$]NaI was much higher than the control solution of quantum dots without radioactivity (Fig. 17). When a $^{124}I$ solution was mixed with the quantum dots, the luminescence intensity was increased further at all wavelength regions compared to that of the simple $^{124}I$ solution. A bump at approximately 800 nm in the mixture solution of $^{124}I$ with the quantum dots was also observed, presumably due to the absorption of UV and blue light emitted from Cerenkov radiation followed by fluorescence emission at 800 nm by the quantum dots. The contribution from radioluminescence of the quantum dots was not further characterized. The effect of the bond distance between $^{124}I$ and the quantum dots on the luminescence intensity may be examined systematically by attaching $^{124}I$ directly to the surface of the quantum dots through covalent bonding.

Thus, it was shown that Cerenkov radiation may be employed as an interal light source and the signals and wavelengths may be controlled by attaching a fluorophore such as quantum dots. Also, tissue penetration may be improved therethrough.

<Example 11> Luminescence imaging of C18 TLC plate using transparent glass plate

A $^{131}$I-labeled compound spotted onto a C18 TLC plate was developed with a mobile phase (MeOH : 10% ammonium acetate buffer = 3 : 7), and the plate was dried completely in air. The plate was scanned for 2 minutes using a radio-TLC imaging scanner (AR-2000, Bioscan, USA), and imaged for 1 minute using IVIS 200. The plate was covered with a transparent glass plate (1.2 mm thickness) prior to the optical imaging measurement in order to attain a higher luminescence signal. The relative intensities of the different spots were measured by counting the photons emitted from each selected spot. The percentage of the region of interest (ROI) from the luminescence imaging and the radio-TLC scanning was comparable (Fig. 18).

Thus, it was shown that the luminescence intensity can be significantly improved by covering with a transparent glass plate having a high refractive index. Also, it was shown that a TLC plate can be imaged directly and quantified rapidly using optical imaging, and Cerenkov imaging might be a good adjuvant to radio-TLC scanning, particularly for radionuclides emitting little or no $\gamma$-rays.

<Example 12> Luminescence imaging of lines and words written with $^{32}$P solution using transparent glass plate

First, lines were drawn on white paper with a pencil, and some lines were drawn over the lines with a $^{32}$P solution (1 mCi/100 $\mu$L in water). The luminescence images of the lines were obtained 1 minute before (left) and after (right) covering the paper with a 1.2 mm-thick transparent glass plate (Fig. 19, a)). The words "CERENKOV RADIATION" were written with a $^{32}$P solution (920 $\mu$Ci/100 $\mu$L). The left image was obtained for 1 minute, and the right image was obtained after covering with a 2.58 mm-thick glass cover. The refractive indices of the two glass plates were not measured (Fig. 19, b)). It was shown that the luminescence signals can be significantly enhanced by covering with a transparent glass plate having a high refractive index. This may be helpful in optical imaging based on Cerenkov radiation and autoradiography.

## Claims

1. A radionuclide detection apparatus (400) for detecting the radionuclide labeled at a subject that can be subjected to HPLC and exists in a fluid flowing in a tube (450), comprising: a chamber (440) through which the tube (450) penetrates; a conversion means (430) provided in the chamber (440) and converting the energy of a charged particle emitted from the radionuclide into Cerenkov light; and a light detection means (410) detecting the light converted by the conversion means (430), wherein the tube (450) has a diameter of 0.1-10 mm, has a light transmitting portion (460) made of any transparent material allowing penetration of light and conversion of the energy of the charged particle into light by the conversion means (430), and is formed at a position facing the conversion means (430),
wherein the conversion means (430) is made from a material having refractive index of at least 1.33 through which material light can pass,
wherein the conversion means (430) converts the energy of the charged particle passing through the light transmitting portion (460), and
wherein the radionuclide emits a charged particle having energy with a threshold T satisfying Equation 1 during radioactive decay:

[Equation 1]

$$T\ (\text{keV}) = 511\ [1 / (1 - 1/n^2)^{1/2} - 1],$$

where n is refractive index of a medium.

## Patentansprüche

1. Radionuklid-Detektionsvorrichtung (400) zum Detektieren des Radionuklids, mit welchem ein Subjekt markiert ist, das einer HPLC unterzogen werden kann und in einem in einer Röhre (450) fließenden Fluid vorhanden ist, umfassend: eine Kammer (440), durch die die Röhre (450) hindurchgeht; ein in der Kammer (440) bereitgestelltes und die Energie eines von dem Radionuklid emittierten geladenen Teilchens in Cerenkov-Licht umwandelndes Umwandlungsmittel (430); und ein Lichtdetektionsmittel (410), welches das durch das Umwandlungsmittel (430) umgewandelte Licht detektiert,
wobei die Röhre (450) einen Durchmesser von 0,1-10 mm hat, einen lichtdurchlässigen Abschnitt (460) hat, der aus einem beliebigen transparenten Material besteht, welches das Eindringen von Licht und die Umwandlung der Energie des geladenen Teilchens in Licht durch das Umwandlungsmittel (430) ermöglicht und an einer Position gebildet ist, die dem Umwandlungsmittel (430) gegenüberliegt,
wobei das Umwandlungsmittel (430) aus einem Material mit einem Brechungsindex von mindestens 1,33 hergestellt ist, durch welches Material Licht hindurchtreten kann,
wobei das Umwandlungsmittel (430) die Energie des geladenen Teilchens umwandelt, das durch den lichtdurchlässigen Abschnitt (460) hindurchtritt, und wobei das Radionuklid während des radioaktiven Zerfalls ein geladenes Teilchen mit einer Energie mit einem Schwellenwert T emittiert, der Gleichung 1 erfüllt:

[Gleichung 1]

$$T\ (\mathrm{keV}) = 511\ [1 / (1 - 1/n^2)^{1/2} - 1],$$

wobei n der Brechungsindex eines Mediums ist.

**Revendications**

1. Appareil de détection de radionucléide (400) pour détecter le radionucléide avec lequel un sujet est marqué, qui peut être soumis à une CLHP et existe dans un fluide s'écoulant dans un tube (450), comprenant : une chambre (440) à travers laquelle le tube (450) pénètre ; un moyen de conversion (430) prévu dans la chambre (440) et convertissant l'énergie d'une particule chargée émise depuis le radionucléide en lumière de Tcherenkov ; et un moyen de détection lumineuse (410) détectant la lumière convertie par le moyen de conversion (430),
dans lequel le tube (450) a un diamètre de 0,1 à 10 mm, a une portion de transmission lumineuse (460) réalisée en tout matériau transparent permettant la pénétration de lumière et la conversion de l'énergie de la particule chargée en lumière par le moyen de conversion (430), et est formé à une position tournée vers le moyen de conversion (430),
dans lequel le moyen de conversion (430) est réalisé en un matériau ayant un indice de réfraction d'au moins 1,33, matériau à travers lequel de la lumière peut passer,
dans lequel le moyen de conversion (430) convertit l'énergie de la particule chargée passant à travers la portion de transmission lumineuse (460), et
dans lequel le radionucléide émet une particule chargée ayant de l'énergie avec un seuil T satisfaisant l'Equation 1 au cours de la désintégration radioactive :

[Equation 1]

$$T\ (\mathrm{keV}) = 511\ [1 / (1 - 1/n^2)^{1/2} - 1],$$

où n est l'indice de réfraction d'un milieu.

【Fig. 1】

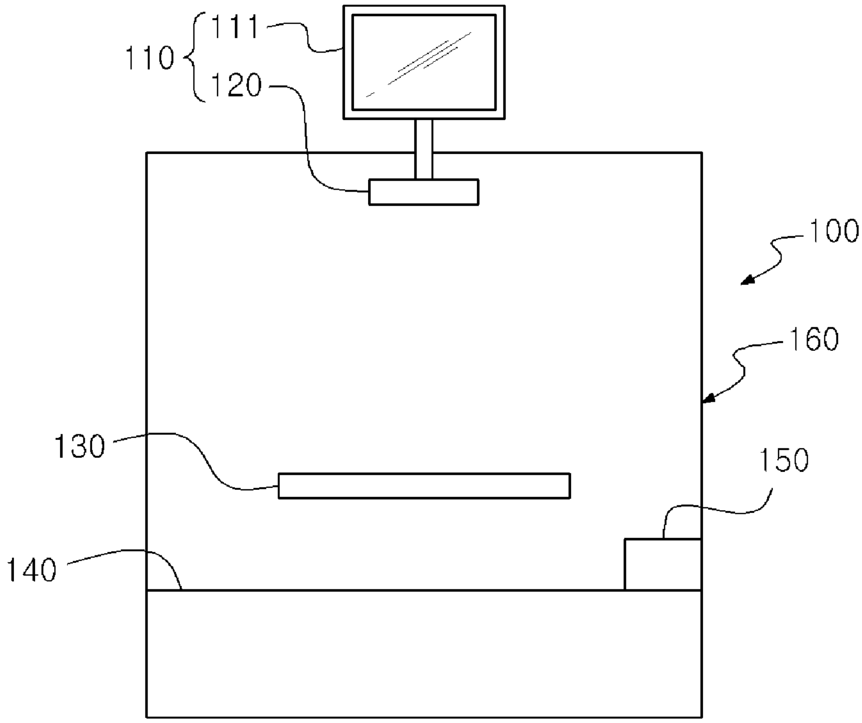


【Fig. 2】

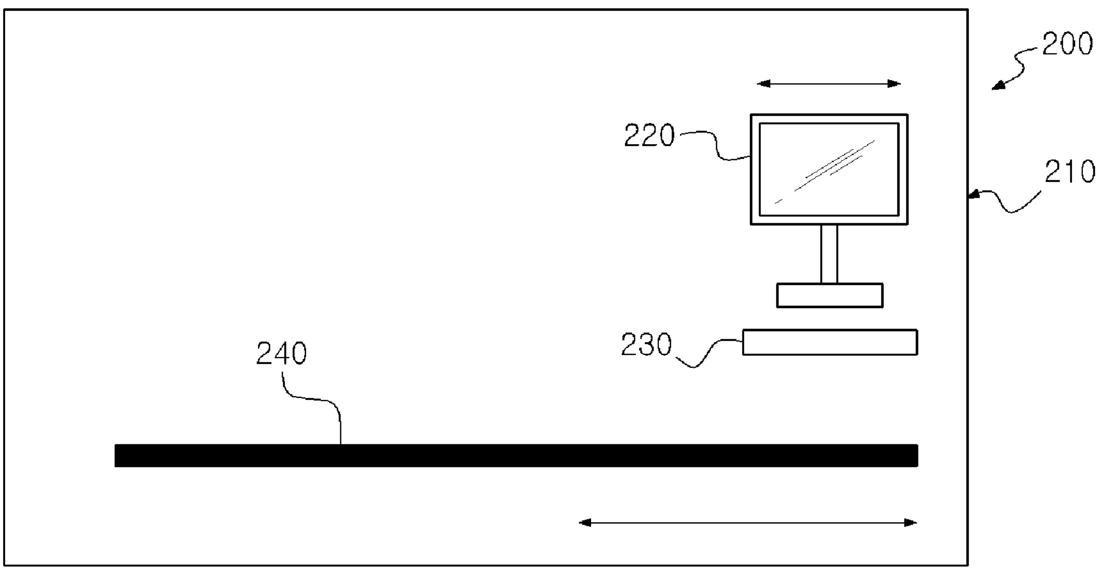

【Fig. 3】

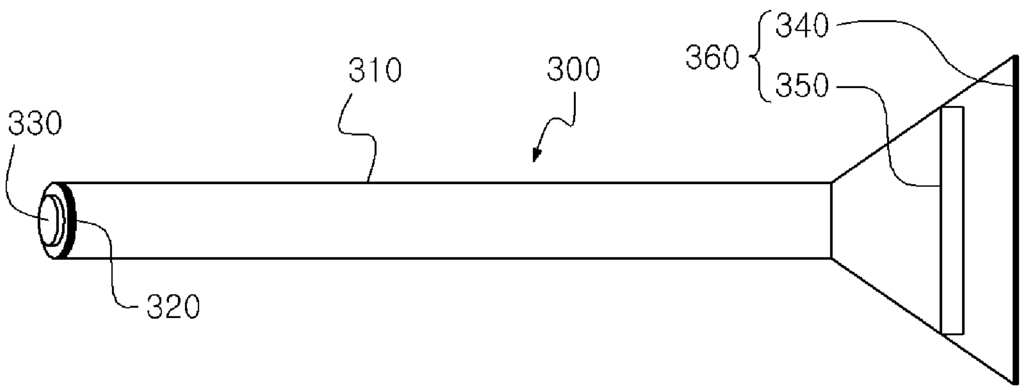


【Fig. 4】

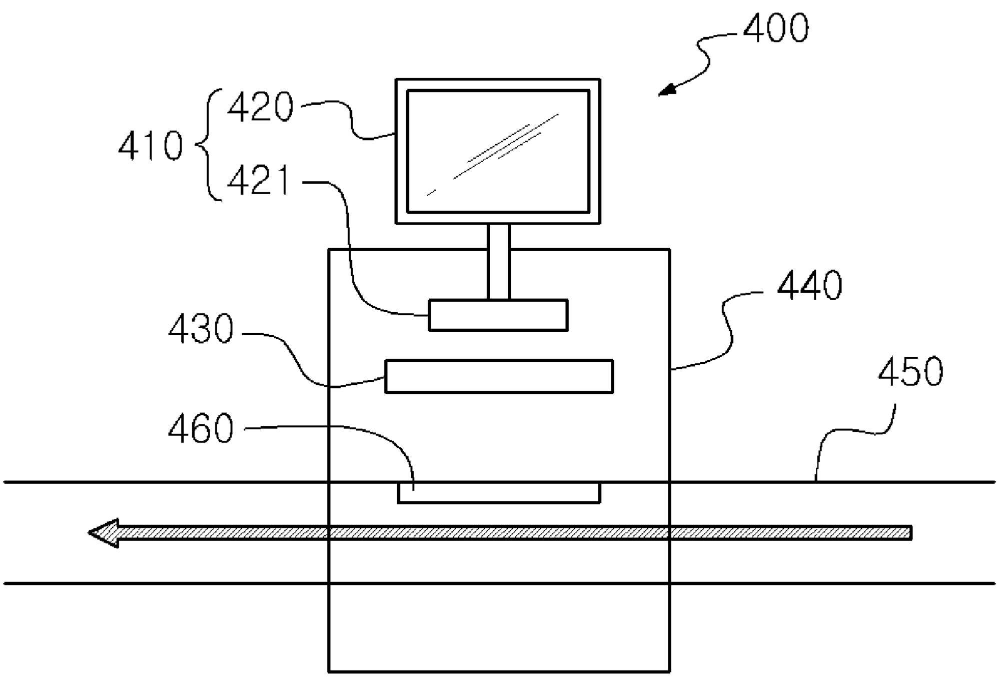

【Fig. 5a】

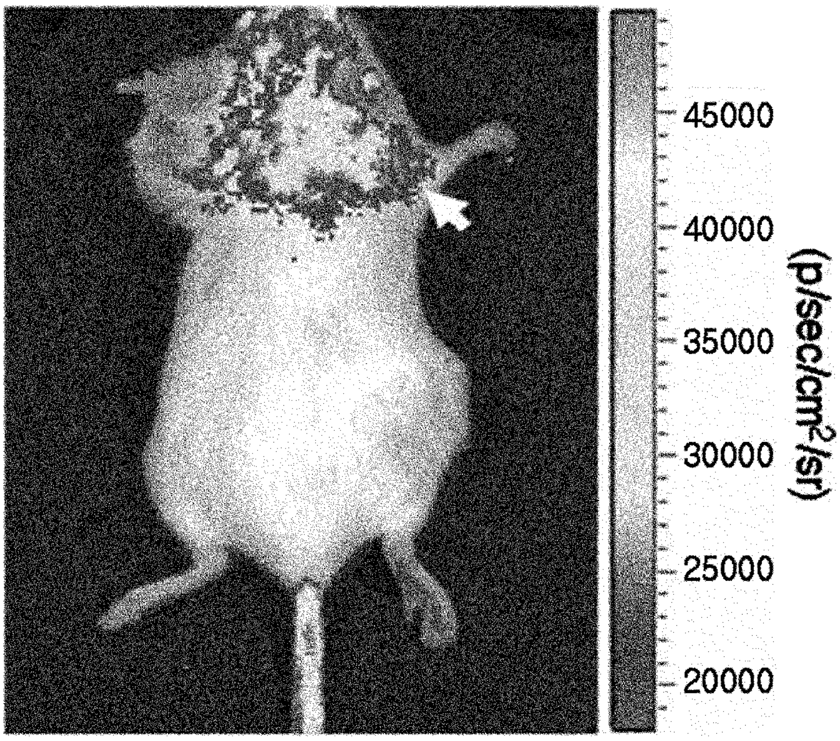
45000
40000
35000
30000
25000
20000
(p/sec/cm²/sr)

【Fig. 5b】

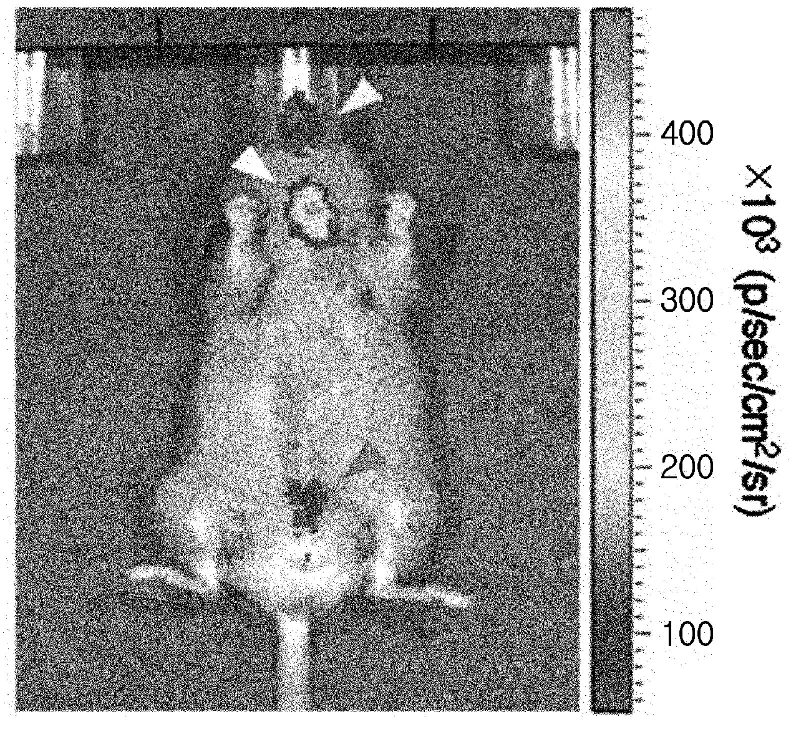
400
300
200
100
×10³ (p/sec/cm²/sr)

【Fig. 6a】

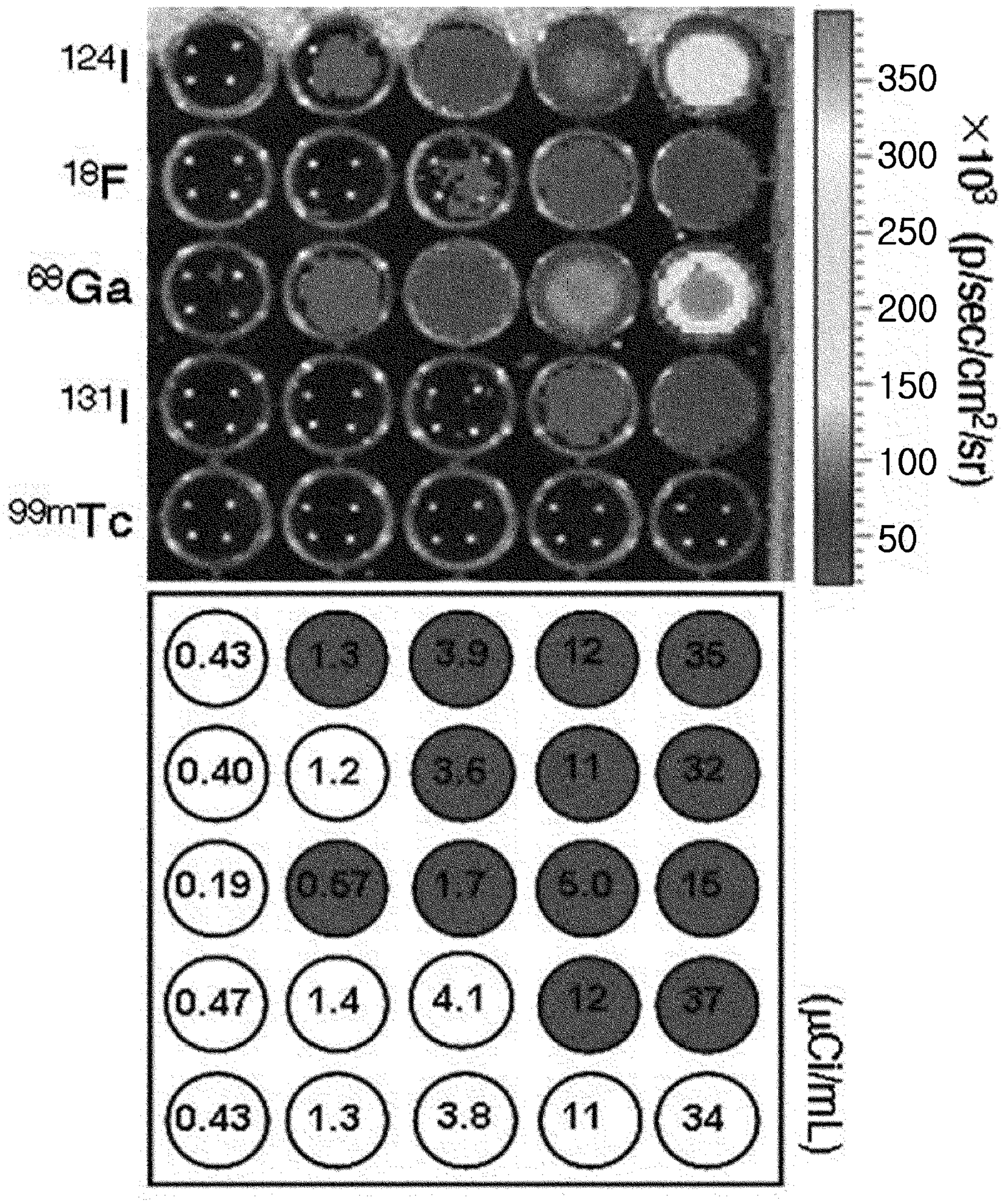
124I
18F
68Ga
131I
99mTc
350
300
250
200
150
100
50
×10³ (p/sec/cm²/sr)
0.43
1.3
3.9
12
35
0.40
1.2
3.6
11
32
0.19
0.57
1.7
5.0
15
0.47
1.4
4.1
12
37
0.43
1.3
3.8
11
34
(μCi/mL)

【Fig. 6b】

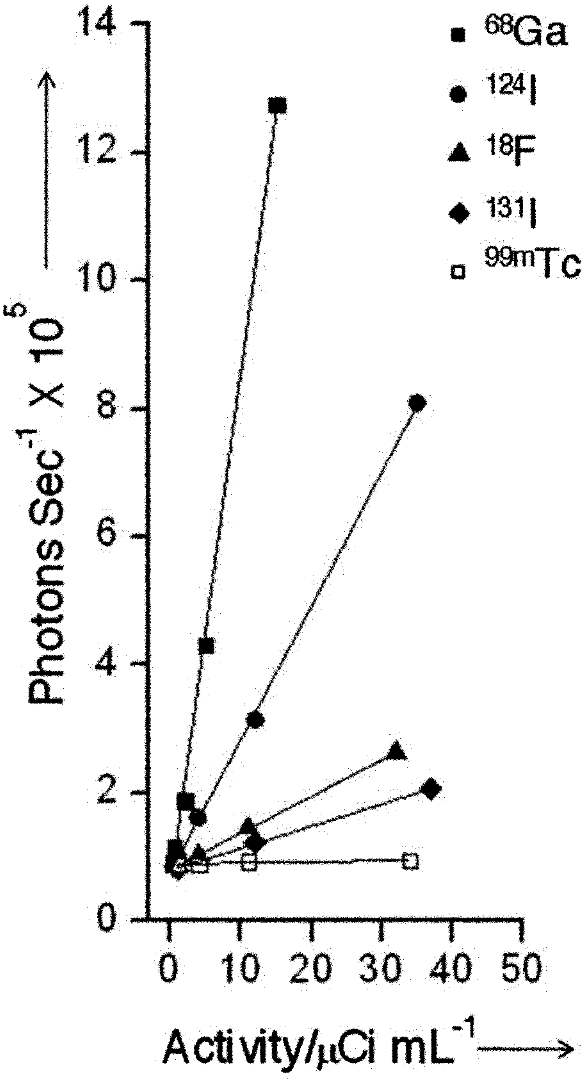

Photons Sec-1 X 10^5
Activity/μCi mL-1
68Ga
124I
18F
131I
99mTc
0
2
4
6
8
10
12
14
0
10
20
30
40
50

【Fig. 6c】

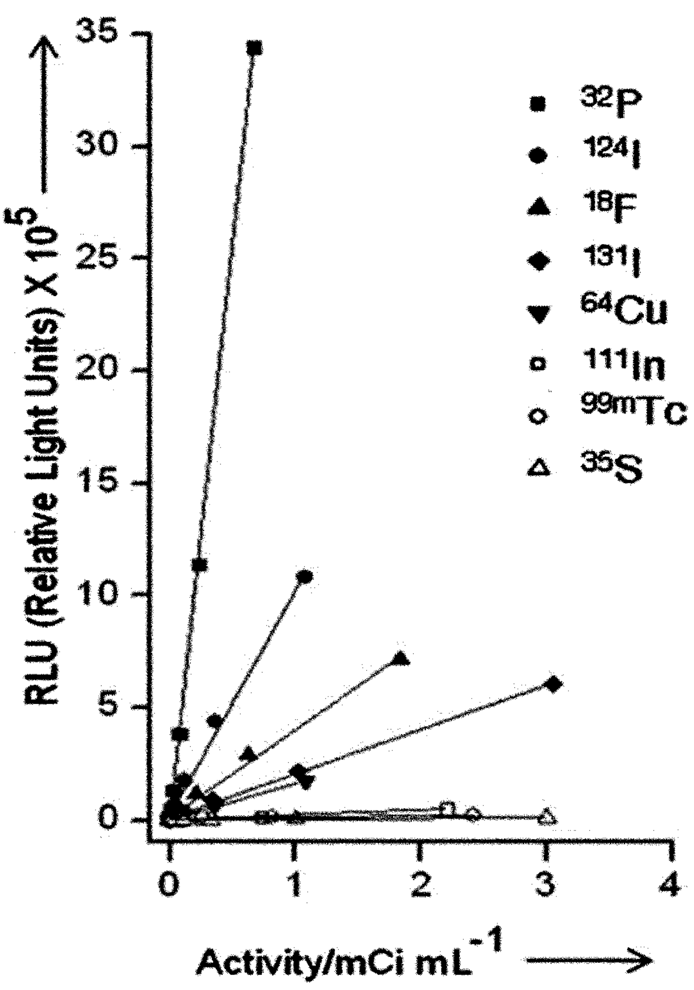
RLU (Relative Light Units) X 10^5
35
30
25
20
15
10
5
0
0
1
2
3
4
Activity/mCi mL^-1
32P
124I
18F
131I
64Cu
111In
99mTc
35S

【Fig. 7】

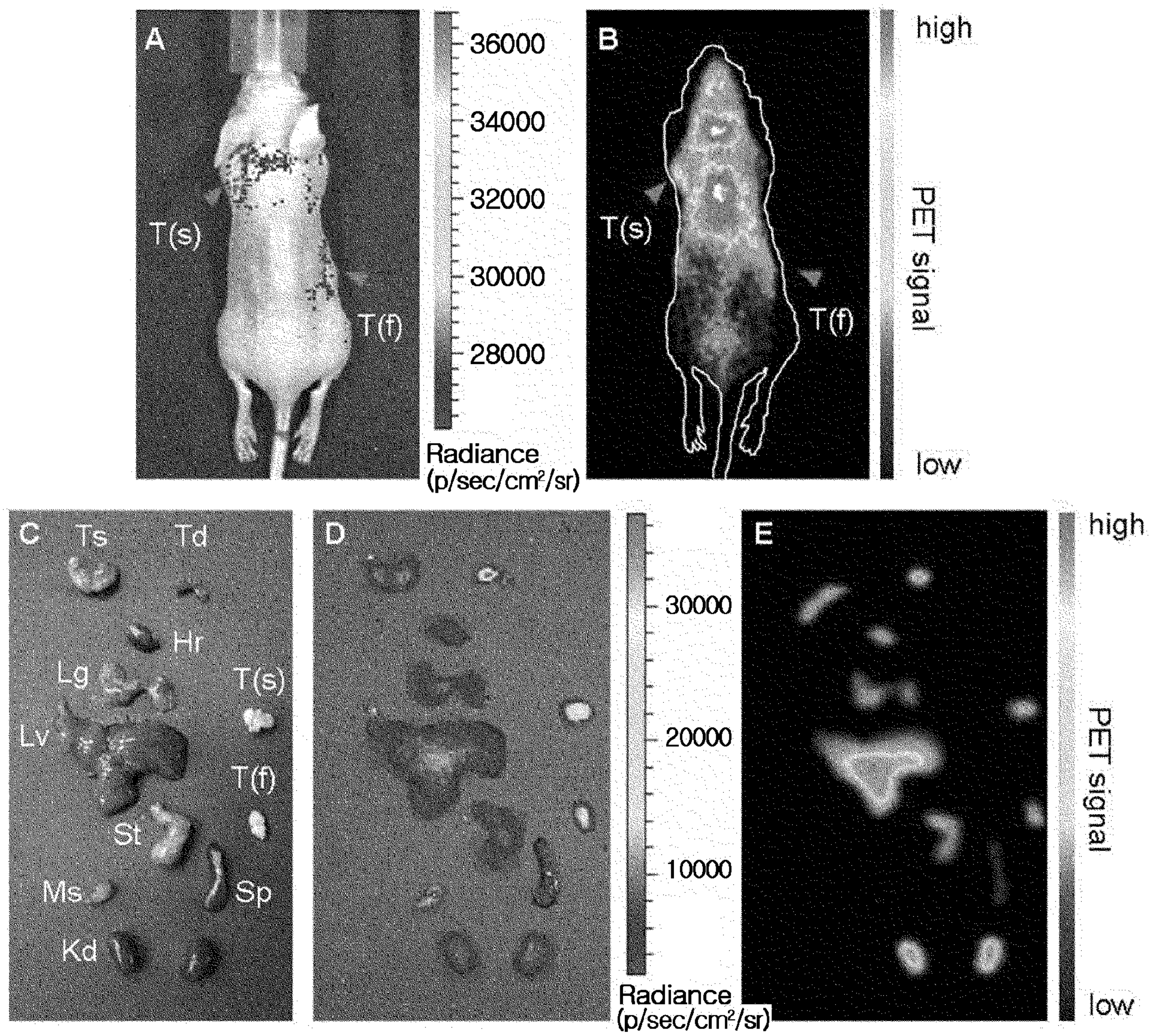
A
T(s)
T(f)
36000
34000
32000
30000
28000
Radiance
(p/sec/cm²/sr)
B
T(s)
T(f)
high
PET signal
low
C
Ts
Td
Hr
Lg
T(s)
Lv
T(f)
St
Ms
Sp
Kd
D
30000
20000
10000
Radiance
(p/sec/cm²/sr)
E
high
PET signal
low

【Fig. 8】

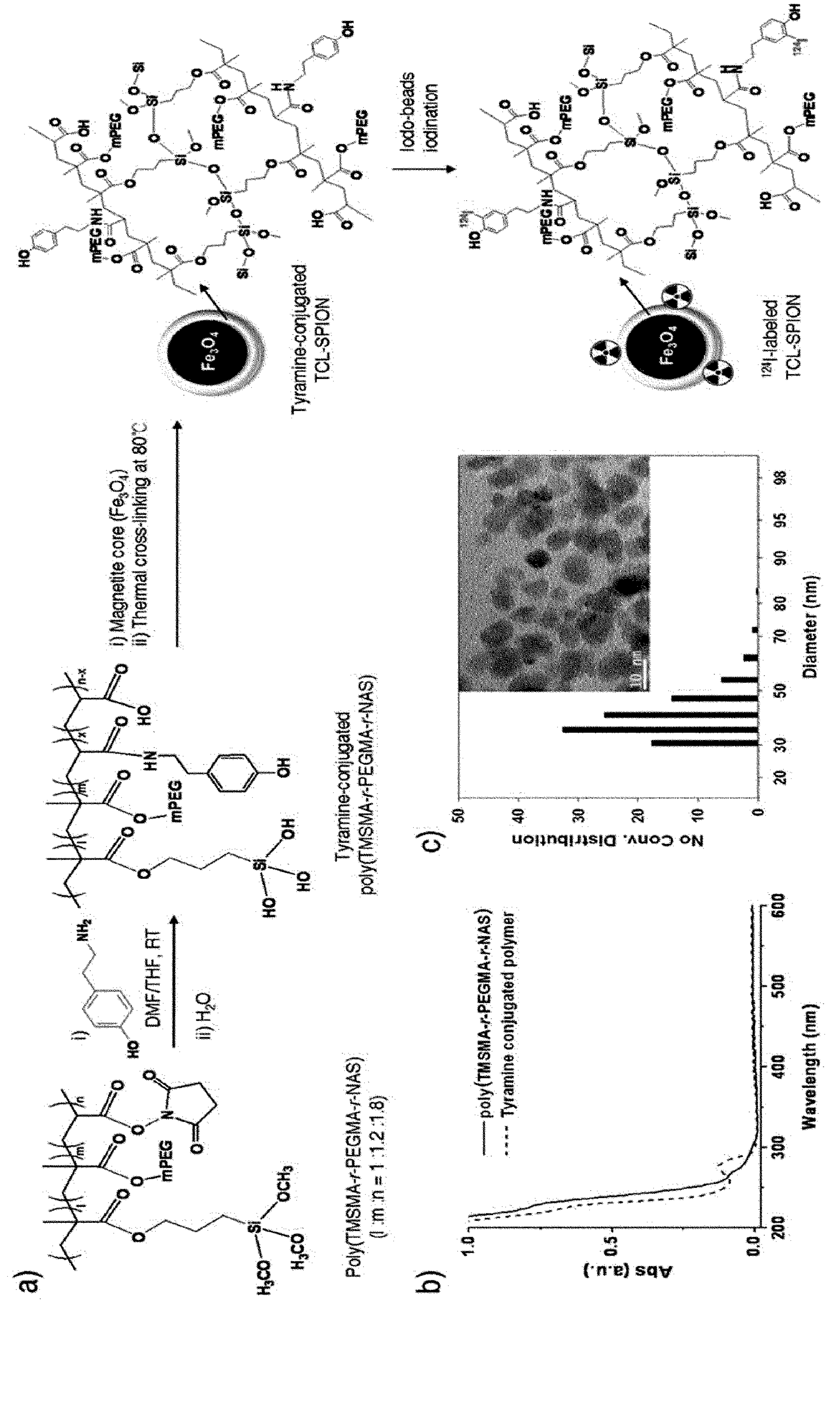
a)
Poly(TMSMA-r-PEGMA-r-NAS)
(l :m :n = 1 :1.2 :1.8)
i)
DMF/THF, RT
ii) H2O
Tyramine-conjugated
poly(TMSMA-r-PEGMA-r-NAS)
i) Magnetite core (Fe3O4)
ii) Thermal cross-linking at 80℃
Tyramine-conjugated
TCL-SPION
Iodo-beads
iodination
124I-labeled
TCL-SPION
b)
Abs (a.u.)
Wavelength (nm)
poly(TMSMA-r-PEGMA-r-NAS)
Tyramine conjugated polymer
c)
No Conv. Distribution
Diameter (nm)
10 nm

【Fig. 9】

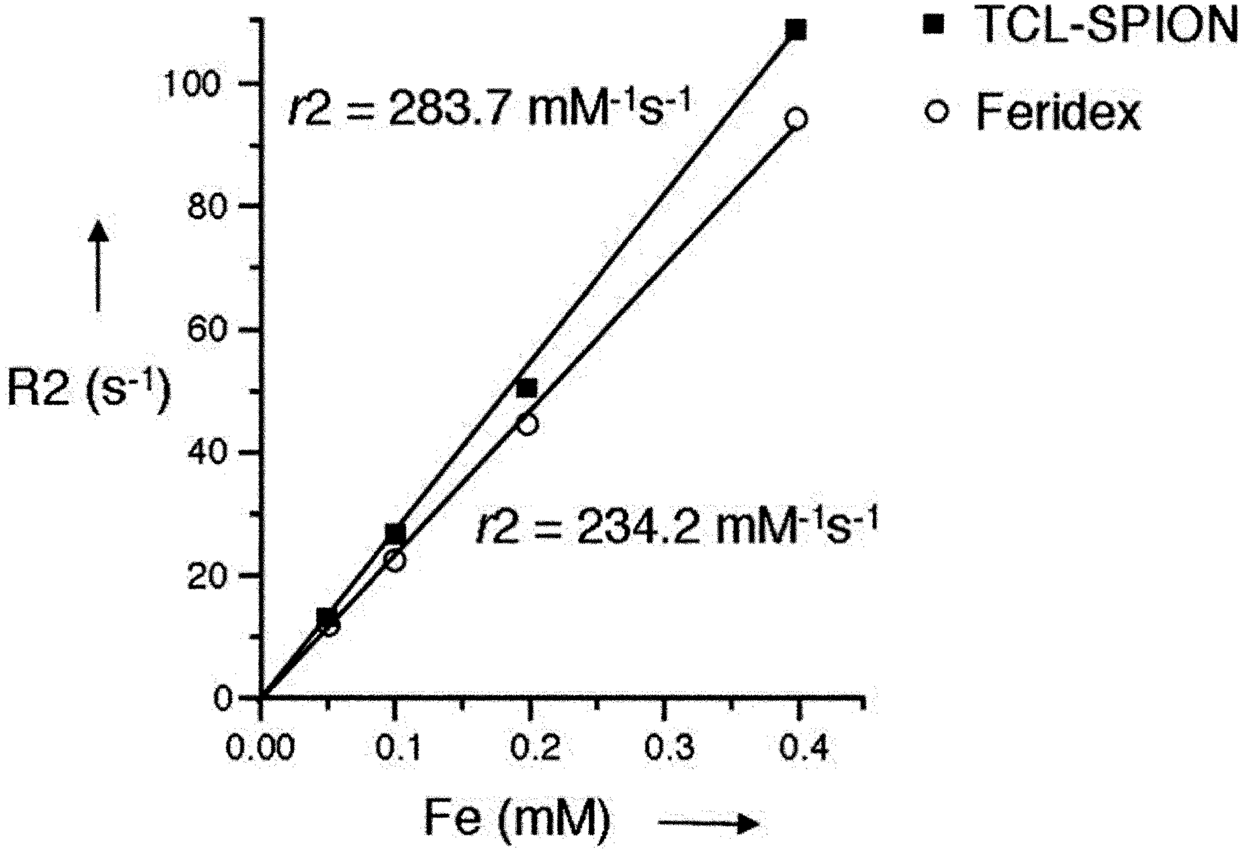
TCL-SPION
Feridex
r2 = 283.7 mM-1s-1
r2 = 234.2 mM-1s-1
R2 (s-1)
100
80
60
40
20
0
0.00
0.1
0.2
0.3
0.4
Fe (mM)

【Fig. 10】

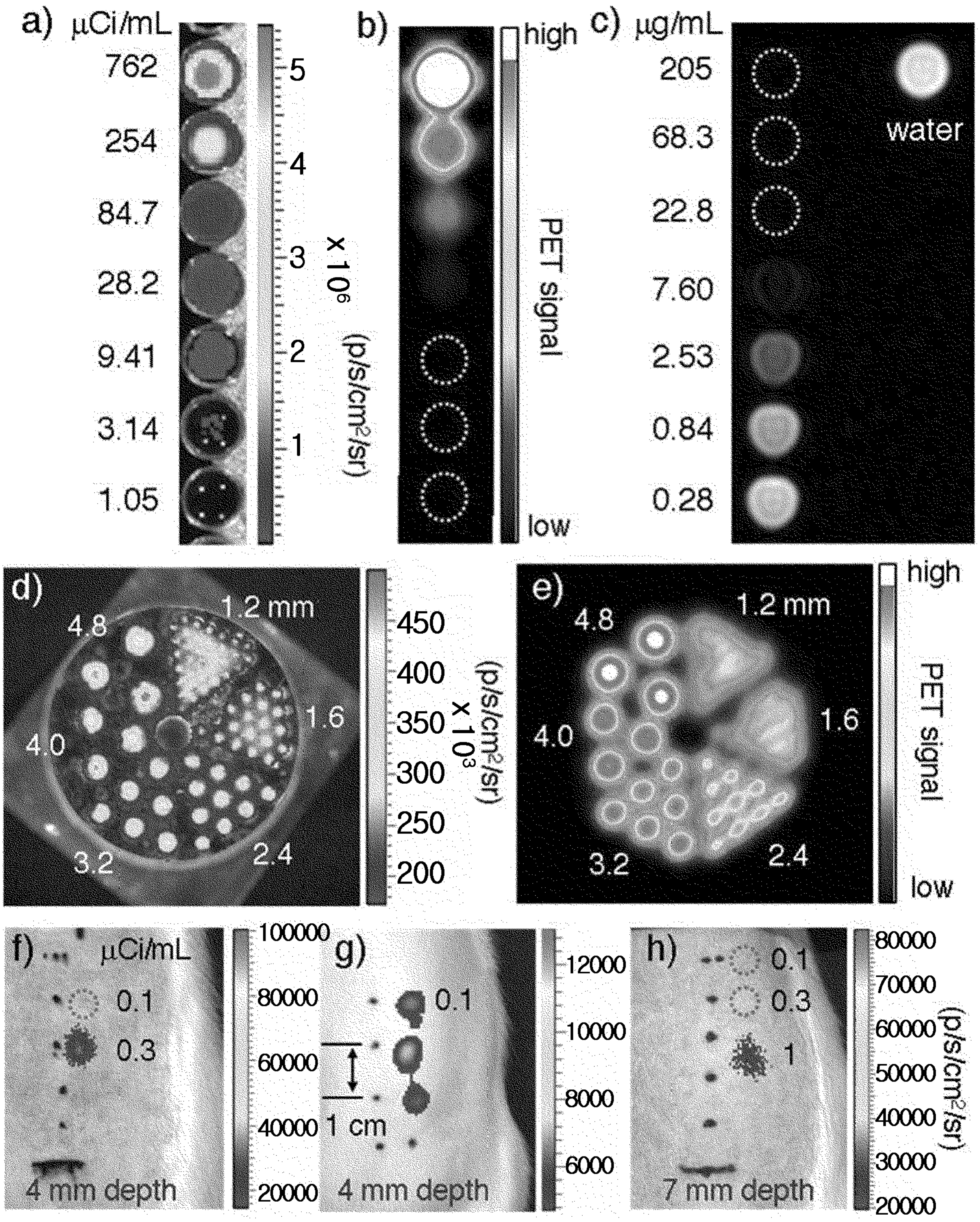

a) μCi/mL
762
254
84.7
28.2
9.41
3.14
1.05
5
4
3
2
1
x 10^6 (p/s/cm^2/sr)
b)
high
PET signal
low
c) μg/mL
205
68.3
22.8
7.60
2.53
0.84
0.28
water
d)
1.2 mm
4.8
1.6
4.0
3.2
2.4
450
400
350
300
250
200
(p/s/cm^2/sr) x10^3
e)
4.8
1.2 mm
1.6
4.0
3.2
2.4
high
PET signal
low
f) μCi/mL
0.1
0.3
4 mm depth
100000
80000
60000
40000
20000
g)
0.1
1 cm
4 mm depth
12000
10000
8000
6000
h)
0.1
0.3
1
7 mm depth
80000
70000
60000
50000
40000
30000
20000
(p/s/cm^2/sr)

【Fig. 11】

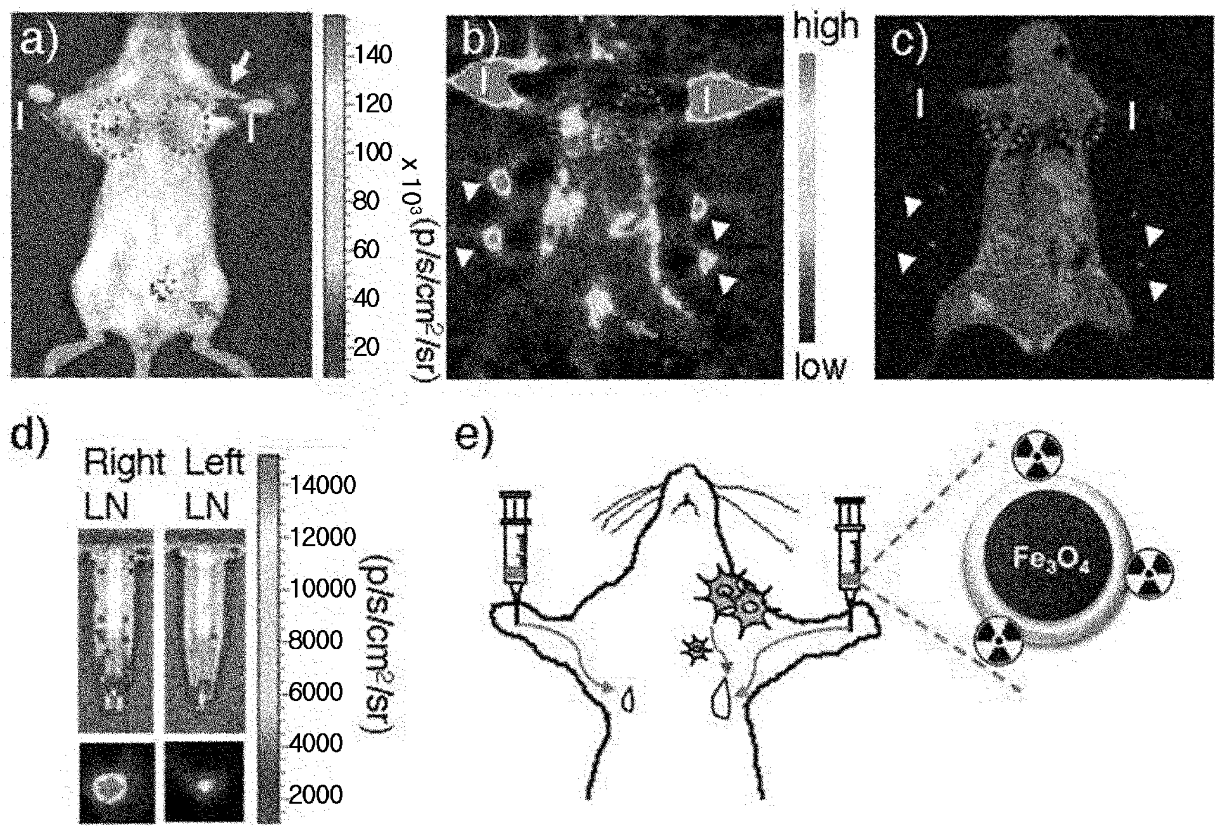
a)
140
120
100
80
60
40
20
x 10³(p/s/cm²/sr)
b)
high
low
c)
d)
Right LN
Left LN
14000
12000
10000
8000
6000
4000
2000
(p/s/cm²/sr)
e)
Fe₃O₄

【Fig. 12】

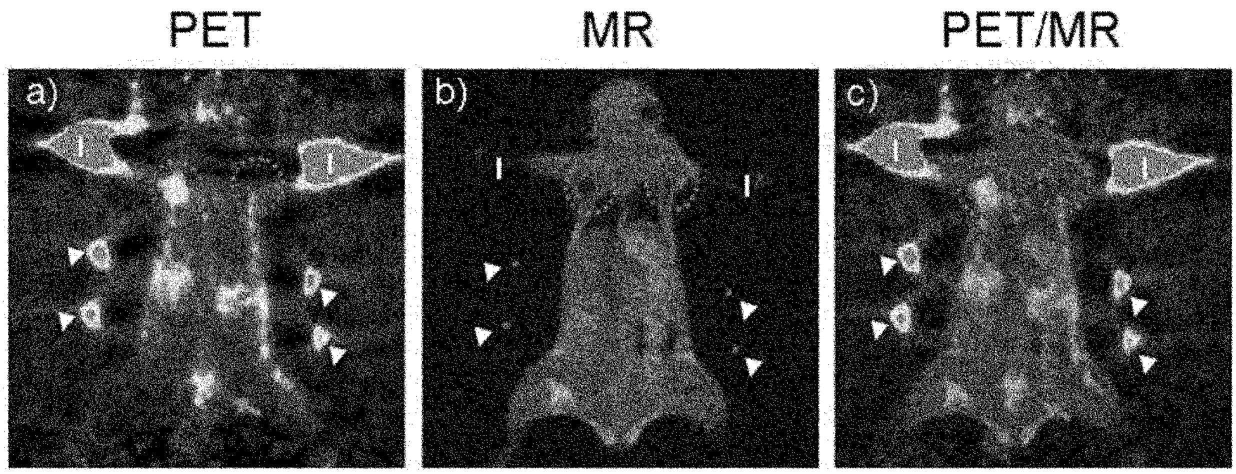
PET
MR
PET/MR
a)
b)
c)

【Fig. 13】

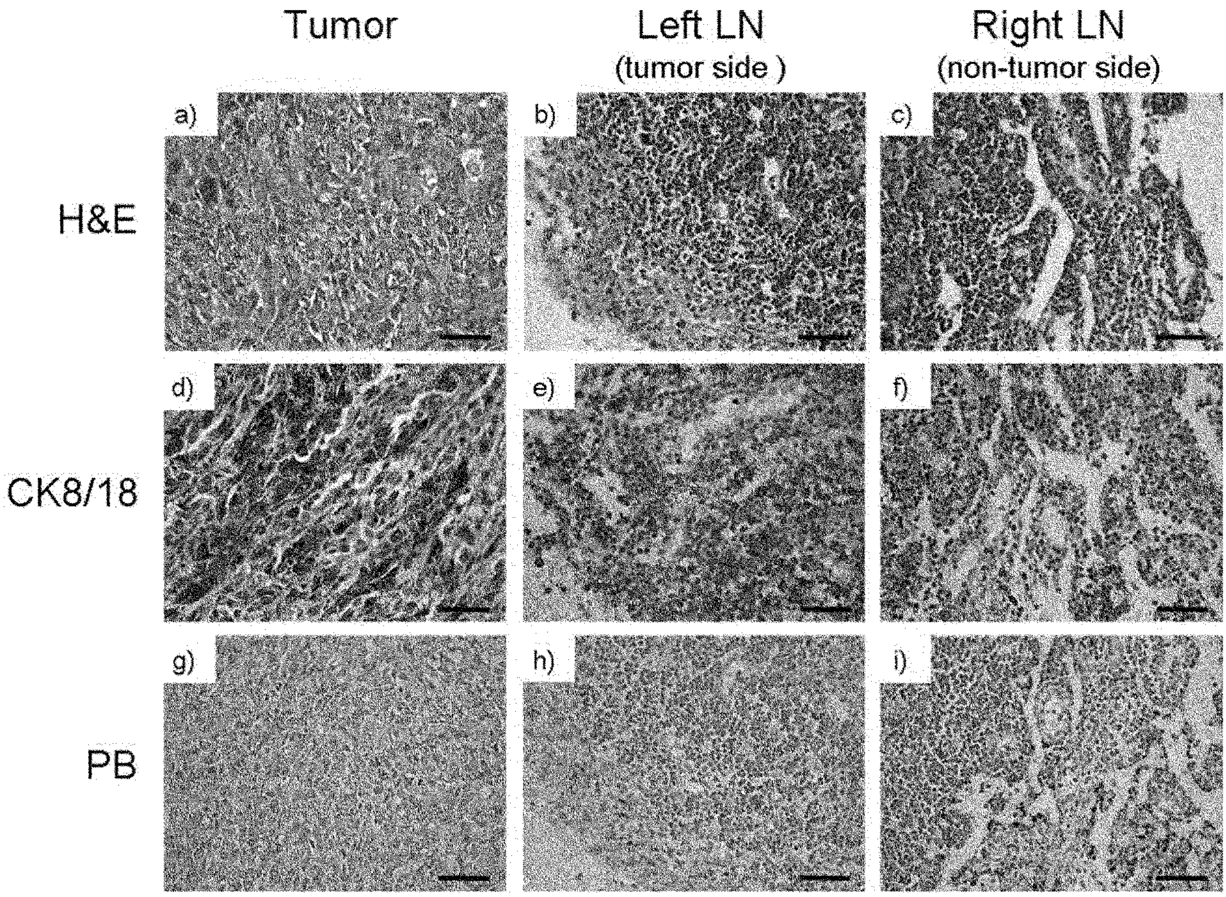
Tumor
Left LN
(tumor side )
Right LN
(non-tumor side)
H&E
CK8/18
PB
a)
b)
c)
d)
e)
f)
g)
h)
i)

【Fig. 14】

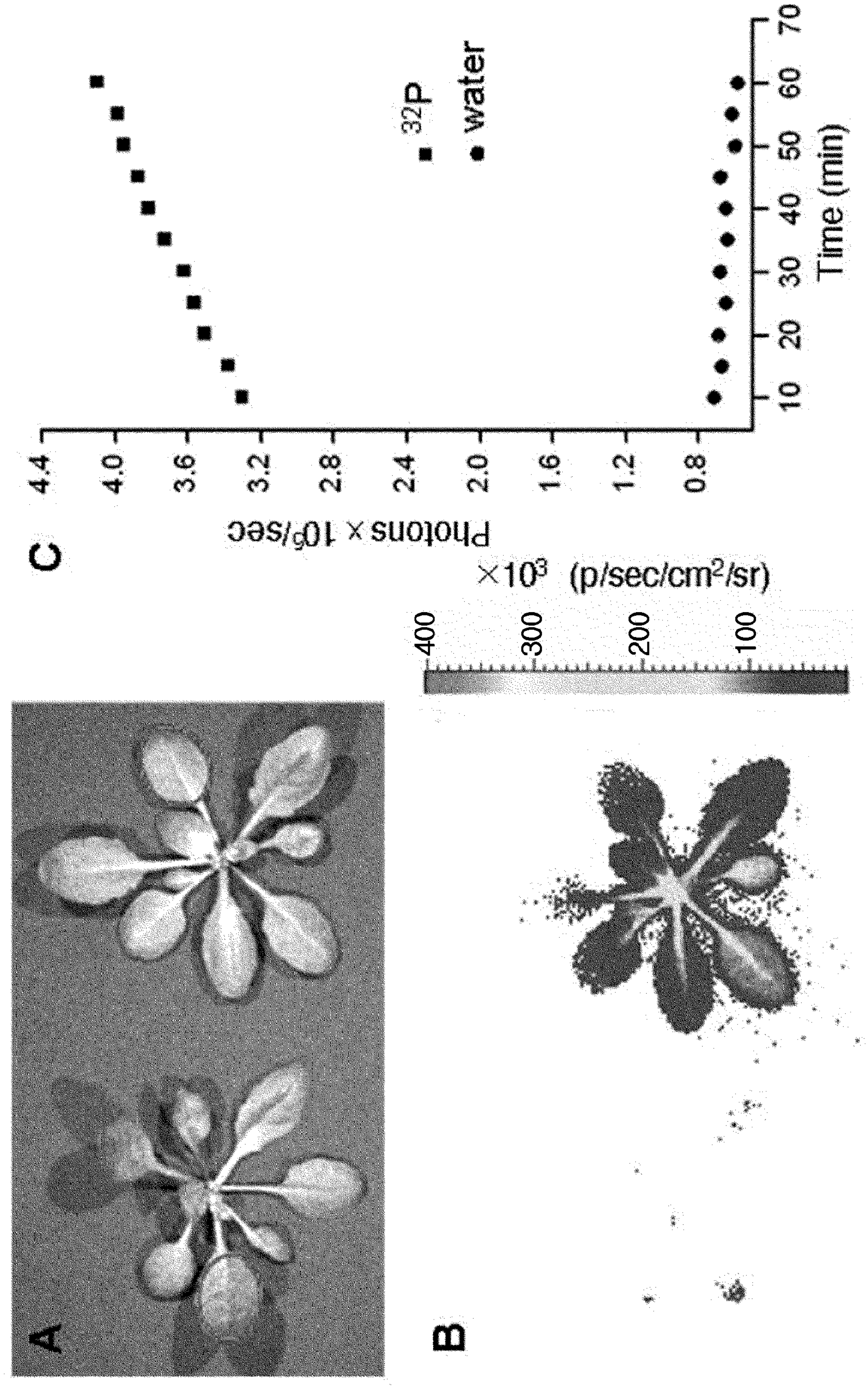
A
B
×10³ (p/sec/cm²/sr)
400
300
200
100
C
Photons × 10⁵/sec
4.4
4.0
3.6
3.2
2.8
2.4
2.0
1.6
1.2
0.8
32P
water
10
20
30
40
50
60
70
Time (min)

【Fig. 15】

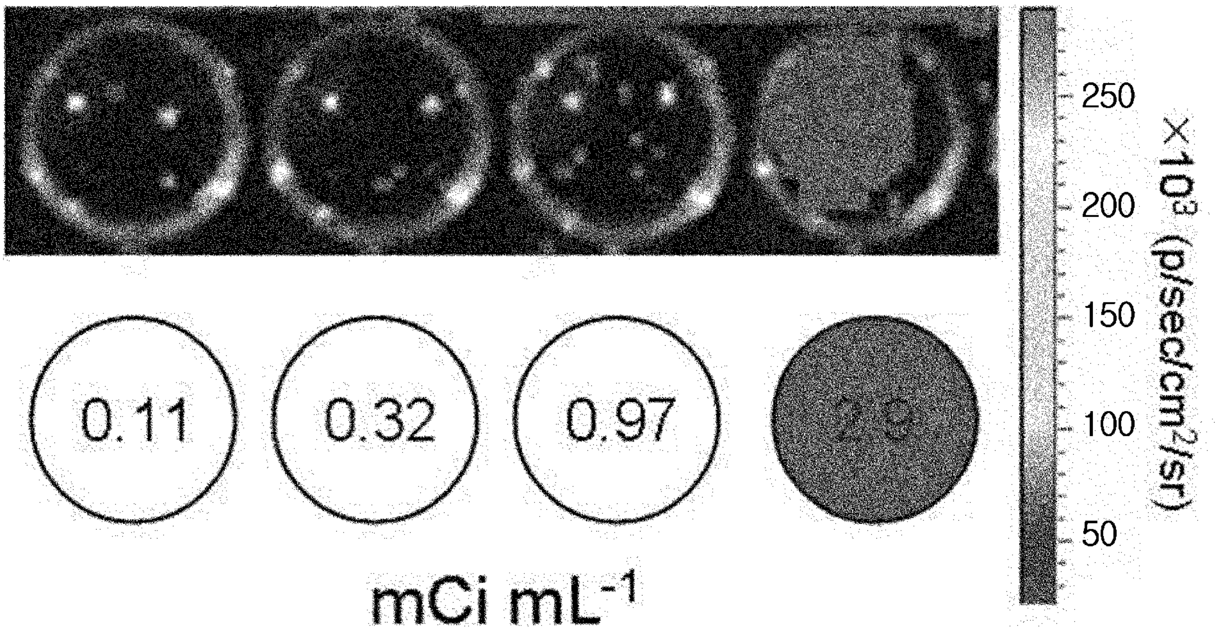

【Fig. 16】

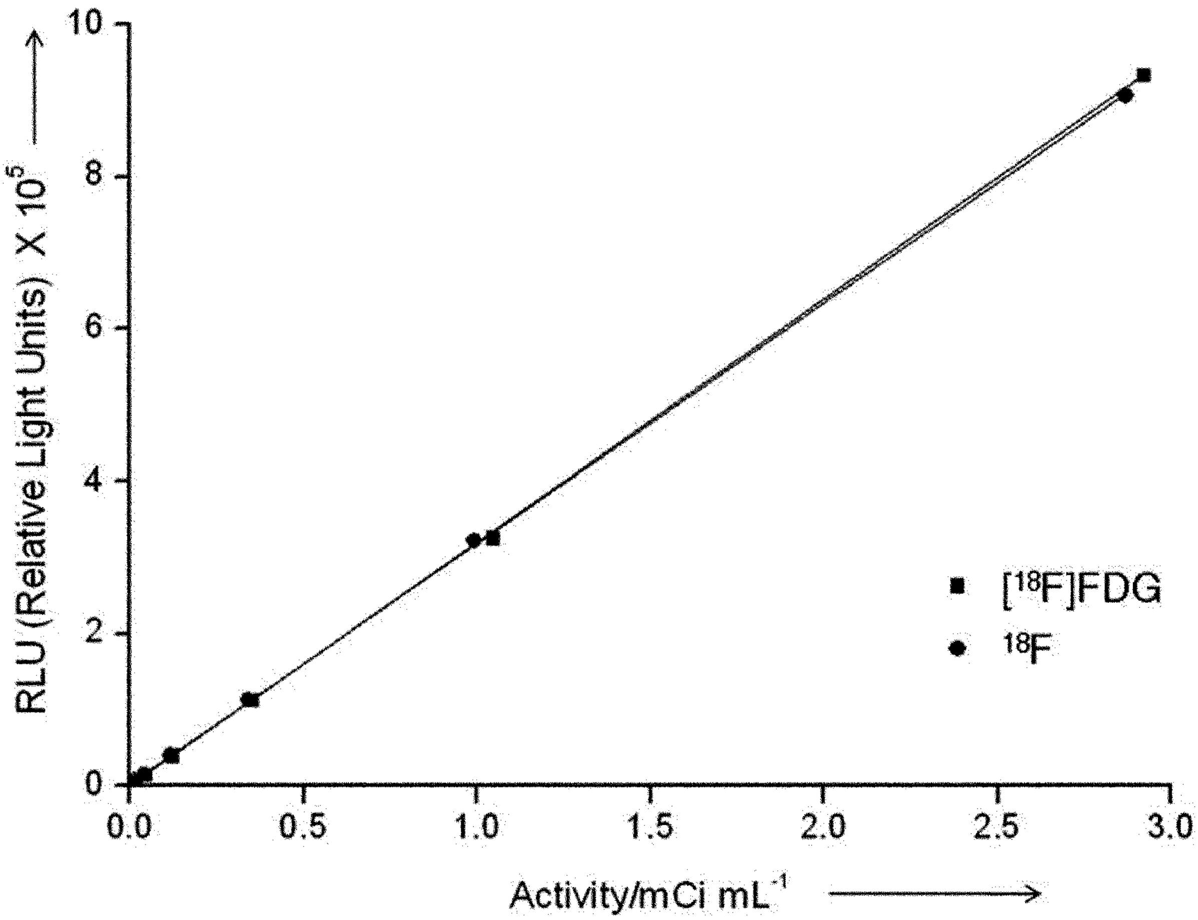
RLU (Relative Light Units) X 10^5
10
8
6
4
2
0
0.0
0.5
1.0
1.5
2.0
2.5
3.0
Activity/mCi mL^-1
[18F]FDG
18F

【Fig. 17】

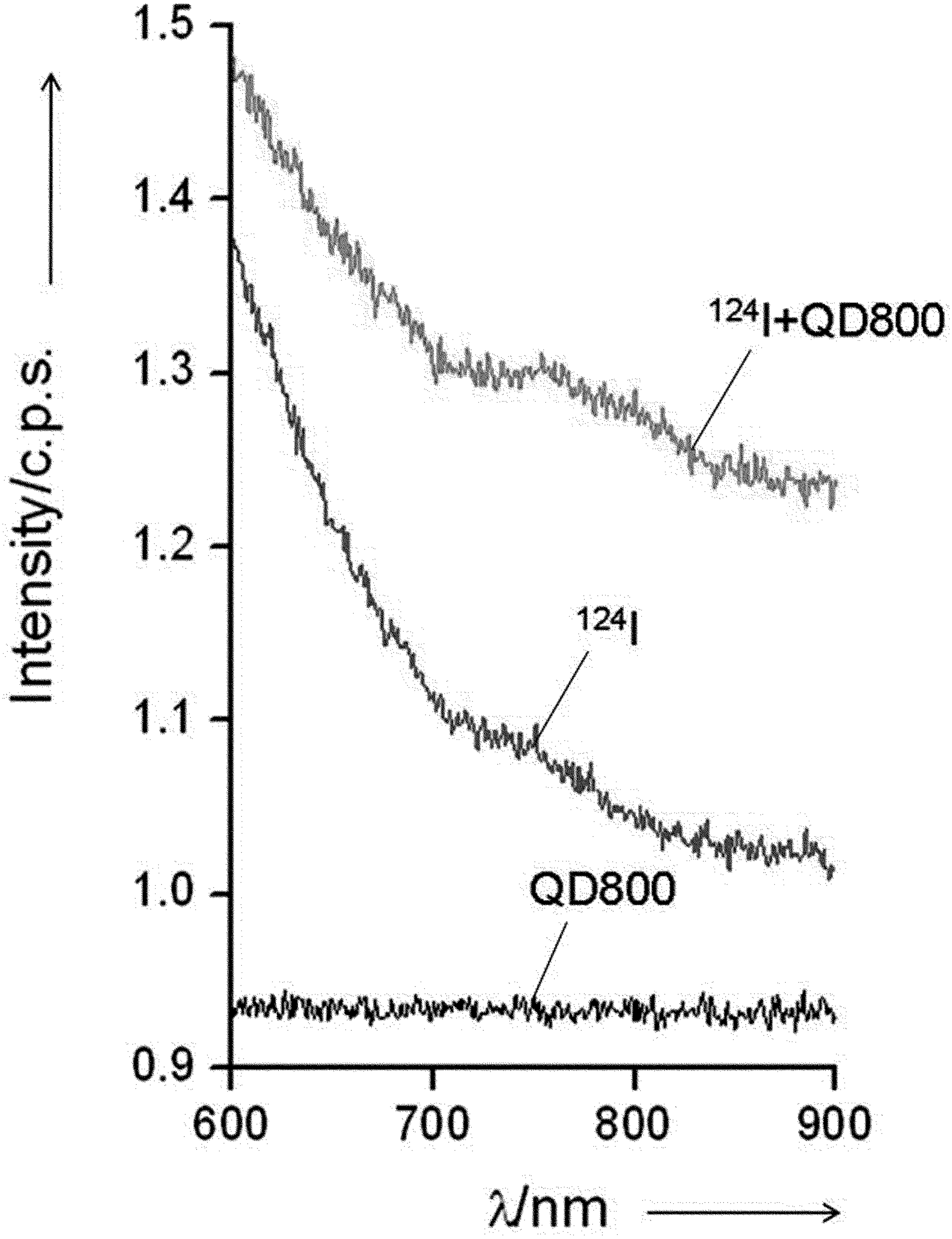
1.5
1.4
1.3
1.2
1.1
1.0
0.9
Intensity/c.p.s.
124I+QD800
124I
QD800
600
700
800
900
λ/nm

【Fig. 18】

A

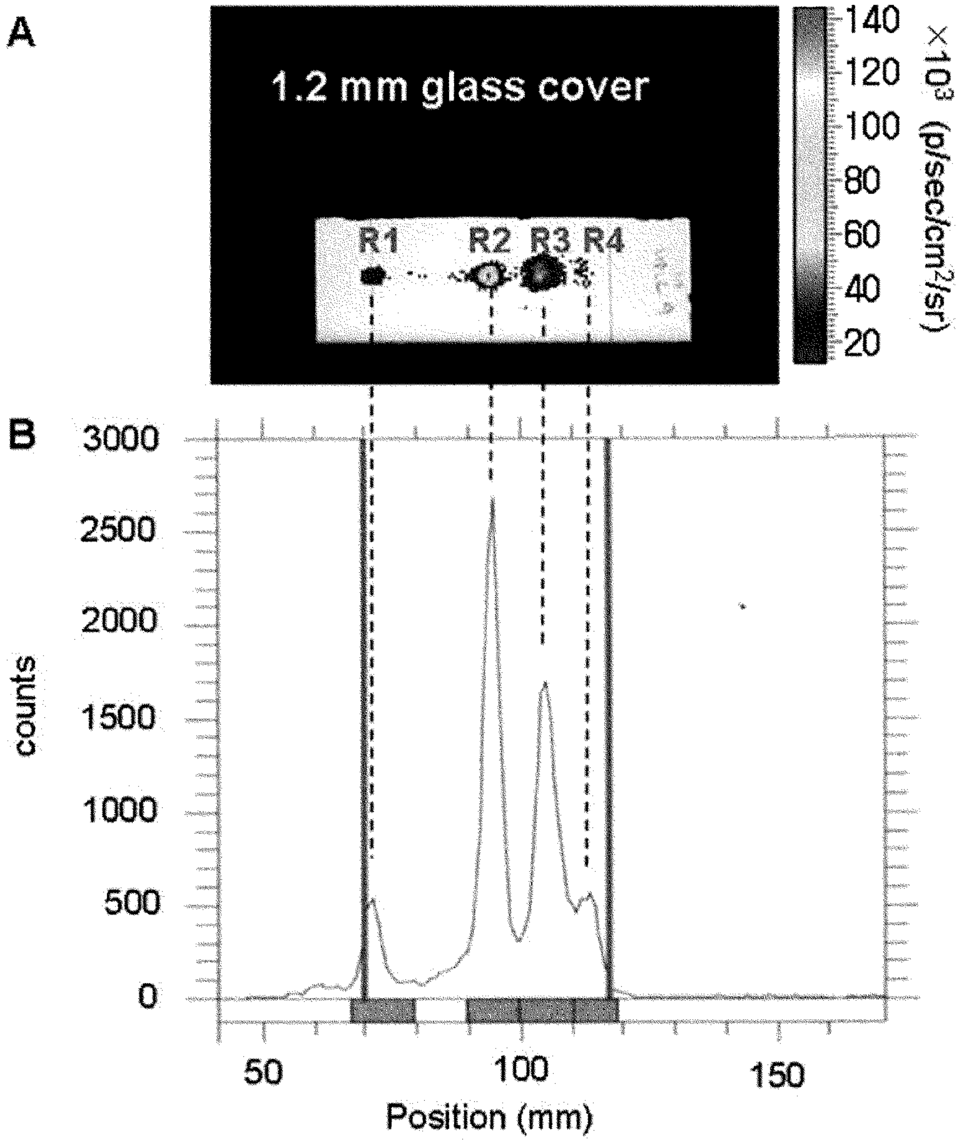


C

| ROI no. | Luminescence image (% of ROI) | radioTLC (% of ROI) |
|---|---|---|
| R1 | 10.2 | 9.6 |
| R2 | 42.4 | 42.4 |
| R3 | 36.7 | 36.2 |
| R4 | 10.7 | 11.8 |

【Fig. 19】

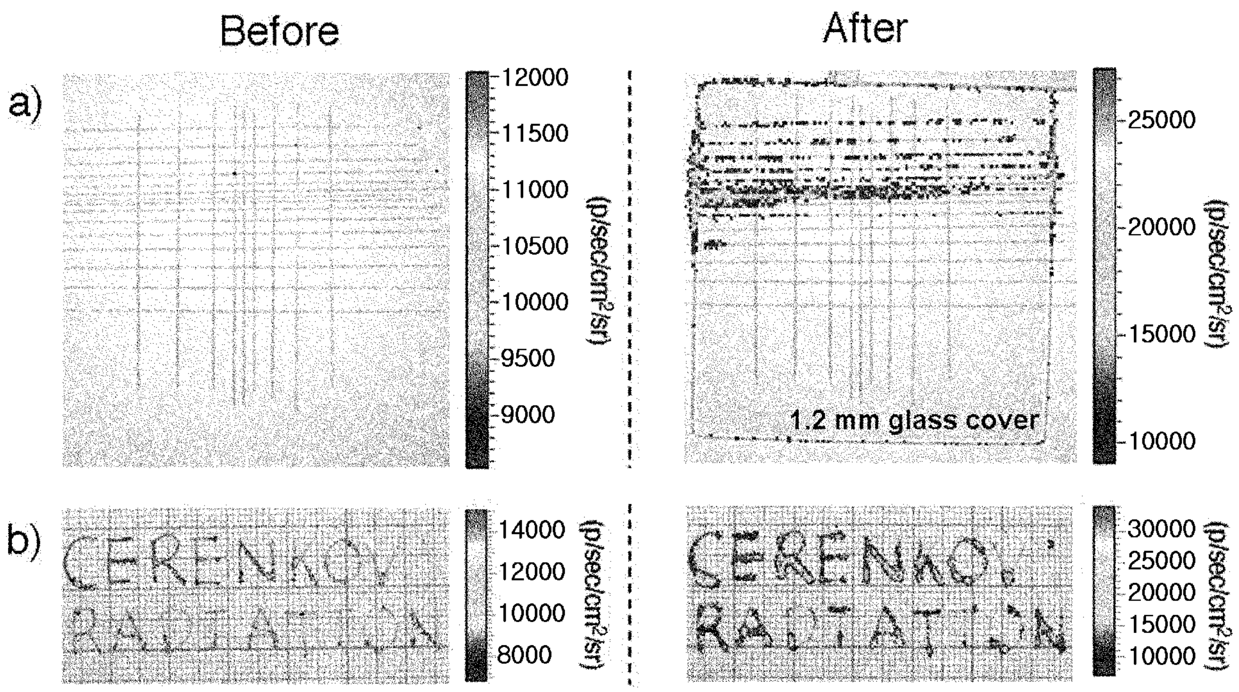
Before
After
a)
b)
12000
11500
11000
10500
10000
9500
9000
(p/sec/cm²/sr)
25000
20000
15000
10000
1.2 mm glass cover
14000
12000
10000
8000
30000
25000
20000
15000
10000
CERENKOV
RADIATION

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20070029030


### Non-patent literature cited in the description


- **ROBERTSON, R. et al.** *Phys Med Biol.,* 2009, vol. 54 (16), N355-365
- The Chemotherapy Source Book. Williams & Wilkens Publishing